# EUROPEAN PATENT APPLICATION

(11) **EP 3 811 929 A1**
(43) Date of publication of application: **28.04.2021**
(21) Application number: 19205138.1
(22) Date of filing: 24.10.2019
(51) Int. Cl.: A61K 9/06, A61K 47/32, A61K 33/20, A61P 31/04, A61P 1/02

(54) **HYPOCHLORITE COMPOSITION AND SYSTEM AND METHOD FOR PREPARING A HYPOCHLORITE COMPOSITION AND USE OF THE SAME**

(71) Applicant: Koninklijke Philips N.V., 5656 AG Eindhoven (NL)
(72) Inventor: MAO, Jingliang, 5656 AE Eindhoven (NL); GODDARD, Gregory Russ, 5656 AE Eindhoven (NL)
(74) Representative: Philips Intellectual Property & Standards

(57) **Abstract**

Provided is a multi-component system, and a method for preparing a hypochlorite composition with increased viscosity with a composition pH in the range of 7 to 12. For example, and preferably, the system and method may be used to provide (hydro)gelled hypochlorite composition. The system or method make use of a first quantity of a first component including hypochlorite or a source of hypochlorite at a pH in the range of 10 to 13.5 preferably 11 to 13.5 and a separate component including a pH induced thickening agent in "dry" form or in dissolved and/or dispersed form at a pH less than 7. The pH induced thickener is capable of increasing the viscosity of a composition upon subjecting it to an increasing pH such as in particular the composition pH in the range of 7 to 12. The pH induced thickener is thus for causing an increase of viscosity in the hypochlorite composition caused by the alkaline composition pH experienced by the pH induced thickener. A pH adjusting (acidic or basic) agent may be present to adjust the composition pH to the desired range. The hypochlorite composition is obtainable by combining amounts of the components in the system.

## Description

### FIELD OF THE INVENTION

This invention relates to systems, kits and methods for preparing a thickened hypochlorite composition from at least two separate components. The invention further relates to use of these systems, kits and thickened hypochlorite compositions for disinfection and sanitization purposes and in particular oral disinfection for prevention and/or treatment of oral infections. The invention also relates to a thickened hypochlorite composition and a method for preparing and use of such composition.

### BACKGROUND OF THE INVENTION

An important topic in oral health care is the prevention and/or treatment of oral infections. Oral infections include for example periodontal and peri-implant diseases which are inflammatory conditions affecting the tissues surrounding the teeth and dental implants. The early stages of periodontal disease, also referred to as gingivitis or gum disease, and of peri-implant disease, also referred to as peri-implant mucositis, are less severe and often reversible when treated in time. The more serious stages of periodontitis and peri-implantitis generally pose more problems and even after treatment often lead to permanent tissue damage and bone loss.

In both oral diseases the inflammation is generally cause by the buildup of pathogenic bacteria in a microbial plaque forming a biofilm around the teeth or implants below the gum line causing damage to the tissue. Therefore, an effective treatment of the diseases involves among others softening and removal of the biofilm and effective elimination of the bacteria.

Currently, most treatments and periodontal maintenance procedures involves use of products in the form of solids, powder, liquid, cream or gels including an antimicrobial agent such as for example hydrogen peroxide, chloramine, or cetylpyridinium chloride or hypochlorite.

One desirable product combines a gel type product with hypochlorite, where the gel provides an extended retention time of the product on the infected area. However, although preformed gels are available, these often suffer from stability issues due to the oxidizing nature of hypochlorite and are difficult to manufacture and handle due to the rheological properties associated with gels.

The known products have disadvantages in that they can be difficult to prepare and handle.

### SUMMARY OF THE INVENTION

The invention as defined by the claims aims to provide improved products at least with regard to one or more of the above disadvantages.

According to an aspect there is provided a system as claimed in claim 1. The inventors have recognized that, considering the aforementioned aim, a careful choice of multiple ingredients and their distribution over at least two separate components of a system of components to be mixed together to prepare a desired hypochlorite composition may be used to fulfill the aim.

The system is a multi-component, multi-part or multi-composition system for preparing a hypochlorite composition having a composition pH in the range of 7 to 12. The system may be a kit of components, parts or compositions. The hypochlorite composition is a hypochlorite containing composition, i.e. a composition that comprises hypochlorite or a source of hypochlorite. It has an increased viscosity and may have the form of a gel and in particular hydrogel. The hypochlorite composition can be obtained by combining or mixing the constituents of the system as elucidated herein after.

The system includes a first quantity of a first component comprising hypochlorite and water. The first component has a first pH in the range of 11 to 13.5. This may provide improved stability of the hypochlorite during storage of the system. A preferred range for this purpose is 11 to 13 or 12 to 13. Other ranges may be used as disclosed hereinafter.

The first component preferably has the form of a solution and/or suspension or dispersion of hypochlorite and/or the source of hypochlorite in water. There may be an optional further solvent and/or dispersion medium.

The system further comprises a second quantity of a second component that is separate from the first component. The second quantity comprises a pH induced thickener capable of thickening a composition including water upon an increase of the pH of the composition from an acidic to an alkaline value. The pH induced thickener is present in at least one of: (i) a solvent and/or dispersion medium free form and (ii) a solvent and/or dispersion medium containing form in which the pH induced thickener is dissolved and/or dispersed at a second pH lower than 7. The second component, when it comprises water as a solvent and/or dispersion medium, preferably has a second pH in the range 2 to 5, more preferably 2 to 4. For example when the pH induced thickener comprises an acrylic co-polymer such as acrylic copolymer based ASE or HASE polymer as disclosed herein after.

Preferably all of the first component, second component and, if any are present, further components, of a system or kit defined or disclosed herein comprise a solvent and/or dispersion medium and they are preferably all aqueous. Mixing of such components is in general easier and/or faster to accomplish with more uniform and faster thickening occurring as compared to a situation where one or more of the ingredients has to be combined in solid state form. Furthermore, improved homogenous distribution and/or mixing of ingredients of each of the system or kit components may be achieved.

The system also may comprise an amount of a pH adjusting agent which is separate from the first quantity of the first component, but may be, but does not need to be, included in the second quantity. This amount of pH adjusting agent is then chosen such that the resulting hypochlorite composition has a composition pH in the desired range of 7 to 12. The agent may be a base or acid and its choice may depending on the pH values of the first and second quantities as well as the final composition pH to be reached. The presence of the pH adjuster agent is optional as there are embodiments that do not need the adjuster agent per se as will become apparent herein below.

The hypochlorite composition can be obtained by combining or mixing at least the first quantity and the second quantity. Optionally, when needed, the amount of pH adjusting agent may be combined and mixed in too.

The pH induced thickener is capable of increasing the viscosity (referred to as thickening) of an aqueous composition when the pH of such aqueous composition (i.e. one that includes water) is raised from an acidic to an alkaline value, e.g. from the second pH to the composition pH. Thus, upon use of the system the thickener ends up in an alkaline environment and causes a substantial thickening of the hypochlorite composition. However, in the system component the pH induced thickener is kept in dry form (without solvent and/or dispersion medium), which is advantageous because many if not all pH induced thickeners comprise polymers in solid state form at standard conditions and solids are more easily handled than viscous compositions and/or is kept in a dissolved and/or dispersed form at acidic pH which prevents the pH induced thickener from exerting its thickening effect in such component.

The system thus provides improved manufacture and handling during use because of its low viscosity or solid state components, while at the same time its use results in a thickened (gel type) hypochlorite composition with all associated advantages.

The pH induced thickeners may be chosen to provide the thickening effect in relatively short time span after or during mixing of constituents of the system as will be elucidated in more detail hereinafter. In some of the embodiments this may be almost instantly or instantaneously, which benefits applications in general and oral applications in particular.

The system has an improved shelf-life, because the hypochlorite is kept at alkaline first pH between 11 and 13.5 at which hypochlorites generally are chemically more stable, while upon use of the system, the hypochlorite composition provides an effective disinfection because of the less alkaline pH range of 7 to 12, preferably 7 to 10 at which the hypochlorite is chemically activated.

In an embodiment, the pH induced thickener comprises or consists of one or more polymers each one comprising monomer residues at least part of which have acidic groups. At least a part of and in most cases the majority of such acidic groups will be in protonated form in the second amount of the second component at the acidic conditions (pH <7 and in deprotonated form at the composition pH in the range of 7 to 12. The deprotonation provides charged sites in the form of conjugated bases of the acidic groups which may engage in polar interactions believed to be responsible for the thickening as explained hereinafter. The acidic groups preferably comprise or consist of one or more groups chosen from carboxylic acid groups, sulfonic acid groups, and phosphoric acid groups. Preferred are carboxylic acid groups and/or phosphoric acid groups. Particularly preferred are carboxylic acid groups. The groups of a particular type (e.g. carboxylic acid) may be all identical or different in the one or more polymers. For example, the difference may arise as a consequence of their binding to the polymer residues via different substituents or groups. This may be used to adjust acidity constants of such groups as known in the art. Many polymers having such groups, and in particular the carboxylic acid groups, are compatible with oral use and/or are environmentally accepted. Such polymers when dispersed or dissolved in the second component may provide the component with the desired second pH without the need for the additional pH adjusting agent. Thus, in some of such embodiments the pH adjusting agent may be omitted. However, it may also be present still for example to adjust the pH of the second composition to a certain desired value or to influence the thickening of this composition.

In an embodiment, the pH induced thickener comprises or consists of one or more alkali-soluble emulsion (ASE) polymers and/or one or more hydrophobically-modified alkali-soluble emulsion (HASE) polymers. Such polymers are generally capable of forming viscous hydrogels with water under alkaline conditions, while they are solids when in dry form or form relatively low viscosity solutions and/or dispersions (e.g. lower than 1000 mPa·s) even in the presence of water under acidic conditions. The ASE and HASE polymers having the acidic groups as defined herein are preferred in this sense. Thus ASE or HASE polymers may provide examples of the one or more polymers each having monomer residues with at least a fraction of their monomers comprising acidic groups.

ASE and HASE polymers are generally capable of dispersing relatively high content of ionic and non-ionic ingredients upon thickening such as the hypochlorite and other ingredients disclosed herein. Furthermore, many of the ASE and HASE polymers such as the acrylic and cellulosic based ones described hereinafter are oxidation resistant and thus may be combined in the hypochlorite composition.

HASE polymers are preferred over ASE polymers due to their improved thickening properties in terms of higher viscosities being attainable at similar concentration and/or faster thickening as well as lower viscosities being attainable at acidic conditions of the second component of the system. These properties may be used to advantage to increase the hypochlorite content in the system and hypochlorite composition and/or to provide higher viscosity hypochlorite compositions. Higher viscosity is sometimes desired for bodily or oral application of the system or hypochlorite composition as the environmental temperature is then almost 40°C and viscosities are known to decrease with increasing temperature. Also mechanical action in the mouth for example by tongue must be resisted by the hypochlorite composition in order to increase resident time in the mouth. HASE polymers may also provide a higher thickening rate due to the additional thickening mechanism which is advantages for direct application of the hypochlorite composition in the oral cavity.

In an embodiment, the pH induced thickener comprises or consist of one or more polymers each having at least a fraction of monomer residues with acidic groups wherein the one or more polymers are acrylic copolymers. An acrylic copolymer is a polymer having monomer residues of one or more acrylic acids (e.g. acrylic acid and/or methacrylic acid or other) and monomer residues of one or more acrylic acid esters or acrylates (e.g. methylacrylate and/or methylmethacrylate and/or or other). Acrylic co-polymers having ester groups with less than 6 carbon atoms provide suitable ASE polymers. Examples of acrylic ASE polymers are the commercially available Acusol™ 810 and Carbomer™ 940F. Acrylic co-polymers where at least part of the monomer residues of acrylic acid esters have a hydrophobic ester group with more than 6 carbon atoms, such as between 6 and 40 carbon atoms provide suitable HASE polymers. One example of an acrylic HASE polymer is the commercially available Acusol™ 820.

In an embodiment within a system as defined herein the pH induced thickener comprises one or more cellulose polymers having residues at least a fraction of which comprise hydroxyl groups. In such case there are no acidic groups in the thickener and other ingredients of compositions having the pH induced thickener may be more compatible with the thickener.

In an embodiment, the pH induced thickener of the second quantity of the second component is entirely in the solvent and/or dispersion medium containing form. Therewith the second component is a second composition in the form of a solution and/or dispersion of pH induced thickener at acidic pH. Preferably the solvent and/or dispersion medium comprises or consists of water. Thus, all of the pH induced thickener is already well distributed in the second quantity. Having a second composition in liquid form makes combination or mixing of system components easier and may result in the composition's ingredients to be more homogeneously mixed compared to a situation where a solid second component needs to be mixed with the first component. Faster viscosity increase may also be achieved with mixing of liquid compositions due to faster more intimate mixing of ingredients. Furthermore, the additional water content may aid the process of increasing of the viscosity in case this process is based on hydrogel formation as will be the case for many of the pH induced thickeners disclosed hereinafter.

In an alternative, the pH induced thickener may be in solvent and/or dispersion medium free form and the second quantity does not comprise any solvent and/or dispersion medium. This dry form is useful when a dilution of ingredients of the first quantity of the first composition such as e.g. the hypochlorite agent is to be reduced or largely prevented upon combining of components. For example, a high concentration hypochlorite composition from a system with substantial dilution would require an even higher concentration of hypochlorite in the first quantity of the first component. Higher concentrations are strongly oxidizing and thus may be disadvantageous in terms of safety and system shelf life.

In certain embodiments, the pH induced thickener may be present at least partly in the aforementioned solvent and/or dispersion medium free form, while there is also solvent and/or dispersion medium present in the first quantity, but separated from the pH induced thickener part. In such embodiment use of the system may involve combining of the solvent and/or dispersion medium with the pH induced thickener for in situ dissolving and/or dispersing the pH induced thickener. This may be advantageous if the pH induced thickener when in dissolved and/or dispersed form still causes a relatively high viscosity (e.g. of higher than 1000 mPa·s) of the second amount of the second component. Such may be the case for some of the ASE polymers such as some of the acrylic co-poloymer ASE polymers.

In an embodiment, the system includes the pH adjusting agent. Preferably such agent is entirely comprised within the second quantity of the second component. This obviates use of additional components for a system. In an embodiment the pH adjusting agent comprises or consists of one or more acidic compounds such as e.g. one or more of: carboxylic acids (e.g. acetic acid and/or citric acid), phosphoric acid, hydrochloric acid and other acid. Preferably the one or more acidic compounds are carboxylic acids and/or phosphoric acid. Such pH adjusting compounds can be used to lower the first pH for example when the pH induced thickener does not itself have acidic groups such as with some of the cellulosic thickeners disclosed herein after. However, even if the pH induced thickener does have acidic groups, the pH adjusting agent may be used to set a desired pH. For example when the acidic groups are weak acidic groups a stronger acidic pH adjusting agent may be used to lower a pH to a desired value or range. Furthermore, the first quantity of the first component may include an amount of (excess) base that is not sufficiently compensated to reach a desired composition pH of the hypochlorite composition by addition of the second quantity to the first quantity without this pH adjusting agent. This may be because the pH induced thickener is not acidic of itself or because its concentration is low.

In another embodiment, the pH adjusting agent is not present in the system at all. In some cases the pH induced thickener is intrinsically acidic as with the aforementioned polymers having acidic groups. In other words, the pH adjusting agent comprises or consists of the pH induced thickener. Thus the pH adjusting agent may comprise acidic groups in the form of carboxylic acid groups, phosphoric acid groups or sulphonic acid groups with a preference for the carboxylic acid groups. Thus the second pH may be already in the desired range due to the acidic nature and amount of the pH induced thickener in the second quantity of second component. Also the pH induced thickener amount and mixing ratio of quantities to be combined may be chosen such that a required composition pH is readily achieved without the need for the pH adjusting agent.

In an embodiment wherein the second quantity includes a solvent and/or dispersion medium, regardless of the pH induced thickener being present in solvent and or dispersion medium free form or not, the ratio of the first quantity to the second quantity is in a range of 10 to 0.1, preferably in the range of 5 to 0.2, or most preferably in the range of 5 to 0.8. The quantities may be measured in volume or weight. This embodiment provides a good balance of necessary quantities to be mixed and may be conveniently used with a multi barrel syringe or the dispenser disclosed herein after.

In an embodiment, the second quantity comprises a solvent and/or dispersion medium with or without the pH induced thickener dissolved and/or dispersed, and the solvent and/or dispersion medium (e.g the second quantity) has a second viscosity lower than 1000 mPa·s, preferably lower than 500 mPa·s and most preferably lower than 100 mPa·s at standard conditions. Such low viscosities improve the manufacture and use of the systems considerably. For example, a viscosity of less than 100 mPa·s is especially desired since such component has a flowability approaching that of water. The pH induced thickeners in the form of HASE polymers disclosed hereinafter may be particularly suitable to provide such low viscosities in the system.

In an embodiment, the amount of pH induced thickener in the second quantity is chosen to cause the hypochlorite composition to have a viscosity higher than 50000 mPa·s, more preferably higher than 100000 mPa·s at standard conditions. Such high viscosities provide stable hypochlorite compositions which is especially useful in oral applications where mechanical forces caused by e.g. tongue may reduce retention time of the hypochlorite composition in the mouth. The pH induced thickeners comprising ASE and in particular HASE polymers are suitable for achieving such values instantly already at modest concentrations in the hypochlorite compositions and often almost instantly as will be elucidated herein after.

In an embodiment, the system comprises one or more additives selected from: a tooth desensitizing agent, a tooth remineralizing agent and a flavoring agent. The presence of any one of such additives may enable tuning of the hypochlorite composition and system for oral use with respect to taste, pain sensation and/or tooth repair. The desensitizing agent and the tooth remineralizing agent may respectively assist in enhancing comfort and protection of teeth during the oral use. The flavoring agent may trigger the sense of taste and/or smell, thereby to provide a better taste sensation during prolonged oral use. The flavoring agent may, for instance, add taste to an otherwise flavorless composition, or may be used to mask any tastes or odors which are less desirable from the point of view of the user of the system or hypochlorite composition.

Any one of these one or more additives may be present in the system in one or more further separate components to not expose them to conditions such as acidic or oxidizing of the first or second component. Preferably they are combined in one further system component or are comprised with the first component and/or the second component. This limits the number of components of a system to 3 or even 2 and thus provides for a simpler system. Preferably they are not present in the first component, but for example are present in the second component. In such case they are not exposed to an oxidizing environment of the first component that may deteriorate their shelf life.

In embodiments the desensitizing agent comprises potassium ions and/or a source of potassium ions such as a potassium salt. Potassium ions are believed to desensitize nerve fibers blocking the neural transmission. For example potassium ions and/or a potassium ion source may be present in the first component and/or the second component. When present in the first component, they may be present in the form of e.g. potassium hydroxide, carbonate or potassium nitrate.

In an embodiment the desensitizing agent comprises or consists of one or more compounds for plugging dental tubules. For example, such compounds may be any one of: Sodium fluoride, Stannous fluoride, Strontium chloride, Silver diamine fluoride, Potassium oxalate, Calcium or strontium phosphate, Calcium or strontium carbonate. Most of these such as the phosphates and carbonates may also serve as remineralization agents.

In an embodiment, the system comprises calcium ions or a source of calcium ions such as a calcium salt and/or strontium ions or a source of strontium ions such as a strontium salt and monobasic, and/or dibasic and/or tribasic phosphoric acid ions (phosphoric ions) or a source of such ions. Preferably the calcium ions, strontium ions or their sources are comprised within the second component and then preferably in a form (e.g. in chloride or nitrate salts) soluble in an aqueous component. The phosphoric ions or their source are preferably comprised within the first component. Separation of the phosphoric ions and their sources from the calcium or strontium ions or their sources may prevent the crystallization and precipitation of calcium or strontium phosphates during shelf life of the system, while upon mixing of the components upon use of the system, they will become combined to form calcium phosphate (CP) and/or strontium phosphate (SP) in crystalline or preferably amorphous (ACP and ASP) form in the hypochlorite composition. The calcium and strontium phosphates may serve as agents of remineralization or desensitization.

A suitable flavoring agent may, for instance, include natural peppermint oil, and or saccharine which may provide the user with a sense of cleanliness and freshness when using the system, particularly when the composition is applied to the dental pieces to be used.

In an embodiment the system comprises a multi-compartment syringe comprising: (i) a first compartment containing the first quantity ; and (ii) a second compartment, comprising the second quantity, the system further comprising a mixing compartment attachable to or attached to the multi-compartment syringe for receiving and mixing the first quantity and the second quantity and having an outlet for permitting the resulting hypochlorite composition to exit the syringe upon the use of the syringe.

The syringe comprises plungers for forcing the components out of the respective compartments and into and through the mixing compartment. The plungers may be designed to be operated synchronously for example since they are mechanically connected to each other. The syringe may provide a convenient means of storing, shipping and use of the kit. It may also provide a simple way of ensuring a desired ratio of quantities of the compositions to be mixed to arrive at the desired hypochlorite composition. Such quantities can be those as disclosed herein.

The mixing compartment may have a channel arrangement for mixing the contents of the respective compartments of the syringe. The channel may have fixed mixing blades for this purpose. Such channel arrangement can be for mixing of fluidic compositions such as solutions, emulsions or dispersions, but may also be arranged for mixing of such fluidic compositions with one or more solid compositions. This will depend on the nature of the compositions to be mixed. The inlet may have two, three, four sub-inlets one for each of the compositions to be mixed. The mixing part may be located in the tip of the syringe and may be integrated in the syringe, or attachable or attached to the syringe.

In an embodiment of a particularly simple system, the syringe comprise only two compartments being the first and second compartments. Any of the ingredients of the hypochlorite composition to be made with this embodiment is then present in one or more of the first and second quantities. This provides a simple two-barrel syringe system.

In an embodiment, the system comprises a dispensing system for dispensing the hypochlorite composition which includes a mixing unit, a user interface and a selection module to control the mixing unit. The user interface combined with the selection module provides a dispenser system which, when loaded with components of the system, allows user definition of a dispensed hypochlorite composition. For example the user may need a hypochlorite composition for a particular use (e.g. oral or regular disinfection), or with a particular hypochlorite content in the hypochlorite composition (for mild or strong disinfection); or with a particular alkaline composition pH (lower for oral application) and/or flavor and/or smell of the hypochlorite composition (for oral application); or with a particular viscosity of the hypochlorite composition. Such parameter may then be used to determine mixing ratios of the quantities of the components of the system.

The dispensing system may have a memory accessible by the dispenser system where the memory is for storing data on contents of systems compositions from which data may be retrieved to calculate the required quantities to be mixed in order to arrive at the required user preferences for the particular parameters.

Thus the user interface may enable the user to select parameters which dictate the nature of the composition ultimately dispensed by the system. For example, the consistency of the composition may be controlled by the pH induced thickener included in the composition and/or the pH of the composition. According to the user selections, the selection module may provide an accurate and efficient means of dispensing the user defined desired hypochlorite composition. In particular, the system may, for instance, enable flavor selection, e.g. when the user has opted and input to the system that the composition is intended for oral application. In a simple example, the user may select either to include the flavoring agent in the composition or not. In a more elaborate example, the user may select from different flavoring agents. The user interface may, for instance, take the form of a touchscreen to enable user-friendly parameter selection.

The system compositions may, for example, take the form of a replaceable cartridge for the dispensing system, with the respective components and separate parts, if present, being contained in separate compartments of the cartridge.

According to an aspect, the systems as disclosed herein may be used to provide a hypochlorite composition for use as an orally applicable disinfectant in or for the prevention or treatment of an oral infection.

The hypochlorite composition may comprise a hypochlorite content in the range of 0.01 to 12.5 wt. %, preferably such content is in the range of 0.5 to 8.0 wt. % sodium hypochlorite equivalent. It will be clear that the same ranges hold for the system from which the hypochlorite composition is made, i.e. the entire system has the same content as the hypochlorite composition. The contents of hypochlorite in one or more of the separate compositions are in accordance with component mixing ratios.

According to an aspect, there is provided a use of a system as disclosed herein for preparing a hypochlorite composition having a composition pH in the range of 7 to 12, preferably in the range of 7 to 9.5, by combining or mixing at least the first quantity, the second quantity and optionally the amount of pH adjusting agent. The hypochlorite composition may be an orally applicable disinfectant for use in the prevention or treatment of an oral infection.

According to an aspect there is provided a hypochlorite composition having a composition pH in the range of 7 to 12, preferably in the range of 7 to 9.5, obtainable by or obtained by combining or mixing a first quantity of the first component, a second quantity of the second component and, optionally, an amount of a pH adjusting agent. The first component, the second component and the optional pH adjusting agent are defined as disclosed herein.

The hypochlorite composition preferably has a viscosity higher than 50000 mPa·s, preferably higher than 100000 mPa·s at standard conditions.

The hypochlorite composition preferably is an orally applicable hypochlorite composition. More preferably the hypochlorite composition is one for use as a disinfectant for use in the prevention or treatment of an oral infection.

In a further aspect there is provided a method for preparing a hypochlorite composition having a composition pH in the range of 7 to 12, the method comprising combining:
- a first amount of a first component comprising hypochlorite or a source of hypochlorite and water and having a first pH in the range of 11 to 13.5;
- a second amount of a second component comprising a pH induced thickener capable of thickening a composition upon an increase of the pH of the composition from acidic to alkaline value, the pH induced thickener being present in at least one of:
   - a solvent and/or dispersion medium free form; and
   - a solvent and/or dispersion medium containing form in which the pH induced thickener is dissolved and/or dispersed at a second pH lower than 7, where such combination results in the hypochlorite composition.

Optionally the system further comprises an amount of a pH adjusting agent chosen to cause the hypochlorite composition to have the composition pH and then the method also comprises combining this amount of pH adjusting agent with the first quantity of the first component and the second quantity of the second component.

The method can have a further step of applying the hypochlorite composition to one or more surfaces of an object to be treated, such as for example of a human or animal subject, and/or of an oral cavity or part of an oral cavity of a human or animal subject.

In the system, hypochlorite composition or method, the use as oral disinfectant in the prevention or treatment of an oral infection may comprise treatment of one or more infections such as gingivitis, periodontitis, peri-implant mucositis and peri-implantitis.

The system and method are designed such that the composition pH of a hypochlorite composition is in the range of 7 to 12. The first and second quantities may provide the entire first component and entire second component present in a system. For sanitization a composition pH in the range of 8 to 10 may be used as a less corrosive alternative. When the system is for use on the body of human or animal subjects such as for the oral applications referred to herein, the composition pH is preferably in the range of 7 to 9.5 or in the range of 7 to 9, or more preferably in the range of 8 to 9 to be better body compatible and still provide good effectiveness of oxidizing action and/or provide a hypochlorite composition with sufficient viscosity.

In case the system or hypochlorite composition is used for bodily and in particular oral application all components and constituents of such system or hypochlorite composition are preferably compatible with such use and this means that they are substantially non-toxic and/or or accepted for oral, pharmaceutical or cosmetic use.

Any of the features of systems may be used to define the uses, methods and hypochlorite compositions as disclosed herein and vice versa, and this may provide the concomitant benefits to the systems, uses and methods as disclosed herein.

### BRIEF DESCRIPTION OF THE DRAWINGS

Embodiments and examples of what is claimed are described in more detail and by way of examples with reference to the accompanying schematic drawings, wherein:
FIG. 1 depicts a kit or system according to an embodiment;
FIG. 2 depicts a kit or system according to another embodiment;
FIG. 3 depicts a kit or system according to yet another embodiment;
FIG. 4 depicts a block diagram of a system according to an embodiment;
FIG. 5 depicts a block diagram of a system according to another embodiment; and
FIG. 6 depicts a flowchart of a method according to an embodiment.

### DETAILED DESCRIPTION OF THE EMBODIMENTS

The detailed description and specific examples, while indicating exemplary embodiments of the kits, systems, compositions, methods and uses, are intended for purposes of illustration only and are not intended to limit the scope of what is claimed. These and other features, aspects, and advantages of the claimed kits, systems, methods and uses of will become better understood from the following description, appended claims, and accompanying drawings. The Figs. are schematic and not drawn to scale. The same reference numerals are used throughout the Figures to indicate the same or similar parts.

Before describing examples in detail the meaning of a few terms will be explained.

The pH induced thickener is capable of thickening of a composition upon increasing the pH of that composition. In particular it is capable of thickening of an aqueous composition. It may have other functions too, but it at least provides the thickening property.

The term "thickening" a composition means increasing the viscosity of that composition.

The term "in the range of' as used to indicate a range of values of parameters such as pH, amounts or contents and viscosity values is meant to include the values of the range limits unless otherwise specified. Thus, e.g. a pH in the range of 7 to 12 is meant to specify that the pH may be 7, 12 or in between these values.

The term "composition" refers to an entity having one or more constituents e.g. as in a pure agent or compound or a mixture of agents or compounds.

The term acidic agent refers to one or more compounds that when dissolved or dispersed in neutral water having a pH of 7 causes the water to have a pH lower than 7. It may refer to Bronsted or Arrhenius acid.

The term "agent" may refer to one or more compounds.

The term "solvent" may refer to one or more pure solvents.

The term "dispersion medium" may refer to one or more liquids.

The term "aqueous composition" may refer to a composition having a solvent and/or dispersion medium that comprises at least 20 % by weight (wt. %) of water. Preferably the solvent and/or dispersion medium has at least 30, or 40 wt. % of water. More preferably the water content is at least 50 or 60 or 70 wt. %. In some embodiments the water content is at least 80, 90, or even 95 wt. %. Ultimately the solvent and/or dispersion medium may be only water.

The term "solvent-free" or dispersion medium-free means that there is less than 5 wt. % solvent and/or dispersion medium. Preferably there is less than 1 wt. % of solvent and/or dispersion medium. Most preferably there is substantially no solvent and/or dispersion medium.

The term "hydrophobic group" means a group that is not attracted or that is even repelled by a mass of water. Such group, does not dissolve in water but may form clusters together, forming micelles. A hydrophobic group in general is an apolar group not capable of substantial hydrogen bonding.

The term "dental pieces" includes teeth, caps, crowns, dentures, etc.

When components are specified to be "separate" or "separated" from each other, they are physically separated.

Specification or definition of parameters are with regard to standard conditions unless otherwise specified. Standard conditions means ambient pressure of 1 atmosphere (1 atm) and room temperature (e.g. 20 °C).

Whenever a content is expressed in % by weight (wt. %), conversion of such values into other content units such as moles per weight or mole per volume or vice versa is done using the relevant molar weights and densities as is usual in the art.

Any suitable analytical techniques known in the art may be employed for analyzing chemical compositions such as those defined in this application. For example, determination of a pH of any composition may be done by dipping a suitably calibrated pH probe into the relevant composition, or by titration, or by use of litmus paper or the like. Furthermore, determining a content of hypochlorite agent may be done using for example redox titration methods suitable for such purpose. Determination of type of ions present and/or their contents in compositions may be done using for example spot test methods for tracing types of ions or flame ionization techniques. E.g. phosphorus, sodium, potassium, calcium, strontium and other ions may be determined in this way. Organic molecules may be analyzed using chromatography, NMR, IR, mass spectroscopy or other spectroscopy methods as is usual. Viscosity measurements may be carried out in many ways such as for example with Brookfield viscometer. As long as the same method is used to generate data to be compared, these can all be used to verify whether compositions fulfill a claims definition. Viscosity values mentioned herein refer to Brookfield viscosities measured at 0.6 rpm and standard conditions unless otherwise specified. All such methods are well-known per se and will not be further described herein for the sake of brevity only.

In summary, provided is a multi-component system, and a method for preparing a hypochlorite composition with increased viscosity with a composition pH in the range of 7 to 12. For example, and preferably, the system and method may be used to provide (hydro) gelled hypochlorite composition. The system or method make use of a first quantity of a first component including hypochlorite or a source of hypochlorite at a pH in the range of 10 to 13.5 preferably 11 to 13.5 and a separate component including a pH induced thickening agent in "dry" form or in dissolved and/or dispersed form at a pH less than 7. The pH induced thickener is capable of increasing the viscosity of a composition upon subjecting it to an increasing pH such as in particular the composition pH in the range of 7 to 12. The pH induced thickener is thus for causing an increase of viscosity in the hypochlorite composition caused by the alkaline composition pH experienced by the pH induced thickener. A pH adjusting (acidic or basic) agent may be present to adjust the composition pH to the desired range. The hypochlorite composition is obtainable by combining amounts of the components in the system.

With the system, pre-forming of viscous compositions in a system can be minimized or avoided since the entirety of the pH induced thickener provided in the system is in a state preventing it to exert its thickening property. Therewith any problems associated with producing, storing, transporting and/or dosing may be addressed.

The system comprises at least two mutually separated components. In some embodiments there may be more than two components such as three, or even four mutually separated components. In particular preferred embodiments the system comprises only three or even only two components that include all hypochlorite compositions ingredients. All components of the system together include all ingredients for the hypochlorite composition to be made with the system. The third component may be used for various purposes such as separation of pH adjuster from pH induced thickener and or separate addition of additives to the system as will become apparent herein below. Having less components provides a simpler system which may be advantageous in terms of cost, ease of manufacture and use. A system having only three and preferably only two components may be advantageously used with a three or two-barrel syringe as described herein after.

The hypochlorite and or source of hypochlorite is part of the first component. It is dissolved and/or dispersed in a solvent comprising water. It may be an aqueous component. The pH induced thickener is part of the second component. It may be present in dry form, i.e. without solvent and/or dispersion medium. This may be advantageous if dilution effects upon combination of system components is to be reduced, minimized or prevented. It may also be dissolved and/or dispersed in the second component. Preferably the solvent and/or dispersion medium includes water with or without a further solvent and/or dispersion medium.

In a preferred configuration the first and/or second quantities are both aqueous compositions. The second component may be a solution or an emulsion or dispersion. A dispersion agent and/or surfactant agent may be present if needed. Since the second pH is below 7 substantial viscosity increase will not be caused by the pH induced thickener when in contact with solvent or dispersion medium. Having the pH induced thickener in dissolved and/or dispersed form instead of in dry form may provide improved mixing properties, e.g. more homogeneous and/or more intimate and/or faster and/or may provide easier packaging and handling in the system. The improved mixing may lead to faster thickening and/or more homogeneous viscosity properties of the hypochlorite gel. An increased water content may aid and facilitate swelling of the pH induced thickener as for many if not all of the pH induced thickeners the thickening mechanism is based on water swelling. Good examples of these are the polyacrylic acid based ASE and HASE polymers and the cellulosic polymers described herein after.

Except for the biologically active ingredients for providing the disinfectant function of the systems herein, any other ingredients, including solvents and dispersion media, are preferably substantially non-toxic or even pharmaceutically or cosmetically accepted when the system and hypochlorite composition are to be used for bodily application such as with oral applications.

The solvent or dispersion medium of any one of the components or quantities of components can comprise or consist of water. For example there may be more than any one of the following amounts of water (measured in wt. % of the total weight of the respective composition): 5, 10, 15, 20, 25, 30, 40, 45, 50, 55, 60, 65, 70, 75, 80, 85, 90. The solvent and/or dispersion medium may include other solvents than water such as one or more alcohols such as ethanol, glycerol, propylene glycol or other. Alcohols, generally mix well with water and may improve the solubility of pH induced thickener or pH adjuster or other system ingredients. Also they may improve the disinfectant working of the hypochlorite composition in a synergistic way as they are known to break open cells.

The system, method and hypochlorite composition may be used for disinfection and sanitization such as in household and industrial settings or for bodily disinfection such as with oral disinfection. Each of these application may entail use of specific ranges of quantities of compositions, amounts of ingredients therein, their pH as well as types of ingredients as will become apparent herein after.

It will be readily appreciated by the skilled person that the relative quantities of compositions in a system will be, at least to some degree, dependent on the nature and amounts of the various ingredients present in the respective compositions, the operative consideration being that the final hypochlorite composition should have the composition pH specified in a desired range with a hypochlorite content in a desired specified range and a particular final viscosity in a desired range.

A guide to design of a system may include the following considerations or steps that do not necessarily need to be followed in the described order.

From a desired quantity (volume or weight) of hypochlorite composition the amount of pH induced thickener needed to provide such composition with a desired viscosity is determined. Also, the amounts of hypochlorite and/or its sources and possible pH adjusters, additives etc. are determined. Taking into account any ratios with which quantities of components of a system are mixed to arrive at a particular hypochlorite composition, the amounts of ingredients in the different components of the system can be determined. When appropriate, dilution, occurring upon mixing of system components, is taken into account according to the usual methods known to those skilled in the art. Calculation may be checked by testing the composition and for example a final hypochlorite composition pH may be measured and possibly corrected when not at spec. The correction may be done e.g. by changing (adding or removing) the amount of pH adjusting agent if needed.

The hypochlorite composition and first component comprise hypochlorite. The term hypochlorite is meant to include hypochlorite groups in free or bound form. A source of hypochlorite is any compound that can provide hypochlorite. Preferred hypochlorite compounds include hypochlorous acid or hypochlorite salts which include hypochlorite ions (ClO⁻ or OCl⁻) compensated with cations, but other compounds such as organic hypochlorite esters which include alkyl groups covalently bound to the oxygen atom of a ClO group can also be employed. The source of hypochlorite may comprise or consist of one or more hypochlorite compounds. Preferred hypochlorite salts having cations chosen from the group consisting of: Li⁺, Na⁺, K⁺, Mg²⁺, Ca²⁺, Sr²⁺ and Ba²⁺. Other hypochlorite salts with different cations ions may be used provided such salts are stable in pure or dissolved and/or dispersed form. When used in or on the body they should also be are acceptable for such use. Most preferred hypochlorite compounds are the NaClO, KClO and the Ca(ClO)₂ as these are fairly stable. Examples of hypochlorite esters are methylhypochlorite or t-butylhypochlorite. Preferably such hypochlorite agents are stable in the systems disclosed.

The amount of hypochlorite or source of hypochlorite in any component of a system such as the first component, or composition is expressed in molality, i.e. moles of hypochlorite group/ion per gram of composition (M_{hg}/g). This allows to account for the different molar weights and number of hypochlorite groups per hypochlorite compound for calculation of content based on weight percentages. Thus, e.g. in a composition there may be 0.000672 M_{hg}/g hypochlorite. This translates into 5.0 wt. % of sodium hypochlorite in the composition as sodium hypochlorite has Mw= 74.44 g/mol and one hypochlorite ion per molecule, while it translates to approximately 9.6 wt. % of calcium hypochlorite in the composition, as calciumhypochlorite has Mw=142.98 g/mol and two hypochlorite ions per molecule.

The hypochlorite content in the hypochlorite composition preferably is in the range of 1.34^{∗}10⁻⁷ to 1.68^{∗}10⁻³ M_{hg}/g (0.001 to 12.5 wt. % NaOCl) hypochlorite, where the corresponding weight percentage values for sodium hypochlorite are given between parentheses since this is a preferred source of hypochlorite.

A preferred range may be from of 1.34^{∗}10⁻⁴ to 1.34^{∗}10⁻³ M_{hg}/g (1 to 10 wt. % NaOCl), more preferably from 1.34^{∗}10⁻⁴ to 1.21^{∗}10⁻³ M_{hg}/g (1 to 9 wt. % NaOCl), or from 1.34^{∗}10⁻⁴ to 1.08^{∗}10⁻³ M_{hg}/g (1 to 8 wt. % NaOCl) or from 4.02^{∗}10⁻⁴ to 1.08^{∗}10⁻³ M_{hg}/g (3 to 8 wt. % NaOCl). Such more concentrated hypochlorite gels may be useful for household or industrial disinfection/sanitization.

Other preferred ranges e.g. useful for oral applications, may be from 1.34^{∗}10⁻⁷ to 0.804^{∗}10⁻³ M_{hg}/g (0.001 to 6 wt. % NaOCl). Preferred ranges are from 6.70^{∗}10⁻⁷ to 0.804^{∗}10⁻³ M_{hg}/g (0.005 to 6 wt. % NaOCl) or from 6.70^{∗}10⁻⁶ to 0.804^{∗}10⁻³ M_{hg}/g (0.05 to 6 wt. % NaOCl).

It will be clear that if the total content of hypochlorite agent in a composition will be present in the total quantities of components mixed together to arrive at the hypochlorite composition. This of coarse holds for all system and composition ingredients.

The pH induced thickener comprises or consists of one or more compounds that have chemical groups responsive to the change of pH from less than 7 to a pH in the range of 7 to 12 by invoking an increase of viscosity of the medium they are part of.

The one or more compounds preferably are oligomers or polymers. The polymers may be homo-polymers having a single type of monomer residue or co-polymers having multiple different monomer residues. The polymers may be regular or random co-polymers. The polymers may be ter-polymers. The polymers may be crosslinked or not.

The chemical groups are preferably distributed along at least part of a, but preferably along an entire backbone of a polymer. The chemical groups may be part of the backbone, directly attached to the backbone with a direct chemical bond, or may be attached via spacers of e.g. saturated hydro carbon chain moieties. Preferably such spacer has less than 5 carbon atoms or even less than 3 carbon atoms. Thus a polymer may have monomer residues part of which or all of which comprise the chemical groups.

A particularly suitable class of polymers comprises as the chemical groups acidic groups which depending on their pKa values may be at least partly deprotonated at the pH in the range of 7 to 12. The acidic groups have pKa values such that a larger fraction is deprotonated at pH higher than 7 than at pH below 7. An acidic group is considered fully protonated in a composition of pH equal to or lower than pKa -2. Conversely the group is considered fully deprotonated in a composition of pH equal to or higher than pKa + 2. Preferably the pKa values of the acidic groups are lower than any one of the following values: 7, 6.5, 6, 5.5 or even 5. A pKa that is at most equal to the second pH of the second component may ensure a surplus of acidic groups being in the protonated form, since around 50% of acidic groups will be deprotonated at pH=pKa. The acidic groups can be chosen to have pKa values in the range of: 2 to 7, or 3 to 7, or 4 to 7, or 5 to 7. Preferably in these ranges the upper boundary is 6 or 5. Preferably the acidic groups are all carboxylic acid groups.

The deprotonation of the acidic groups at alkaline pH may result in the aqueous solubility of the polymer being higher at the alkaline pH of 7 to 12 than at the acidic pH of less than 7. The enhanced aqueous solubility may result in a greater degree of swelling of the polymer with solvent (water) molecules, e.g. relative to the degree of swelling at the lower pH. The swelling of the polymer is in turn accompanied by formation of a gel-like composition which has an increased viscosity.

Suitable acidic groups may be sulphonic acid groups, phosphoric acid groups and carboxylic acid groups. A polymer may have a single type or a combination of two or more of such types of groups. Preferably all groups are carboxylic acid groups that have the same or different pKa. For example, carboxylic acid groups attached to saturated carbon spacers have pKa different than those attached to aromatic spacer. Such groups may for example be part of polymers disclosed herein after.

The pH induced thickener may comprise an alkali soluble emulsion (ASE) polymer.

An ASE polymer is capable of forming an alkali-soluble emulsion when dispersed in an aqueous composition at a pH below 7. Without wanting to be bound by theory it is believed that the thickening property of the ASE polymers is based on a non-associative mechanism based on polar repulsive interactions (e.g. charge-charge interactions) between polar groups occurring upon subjecting it to increasing pH values such as above 7.

One group of ASE polymers comprises the aforementioned acidic groups for example in the form of the carboxylic acid groups to provide the polar groups. Such ASE polymers may form solutions or emulsions at acidic pH due to reduced deprotonation of the acidic groups. The solutions or emulsions may have a relatively low viscosity, e.g. lower than 1500 mPa·s. Increasing the pH results in the aforementioned deprotonation and therewith formation of charged groups (conjugated base of the acid group) and concomitant increase of solubility of the polymer which is accompanied by a degree of swelling far greater than that at the acidic pH. This greater degree of swelling causes the increase in the viscosity to values higher than 5000 mPa·s or even higher values as disclosed herein.

A group of polymers having acidic groups and that may provide good ASE polymers are acrylic co-polymers comprising or consisting of monomer residues of at least one acrylic acid and at least one acrylic acid ester (acrylate residue) having an alcohol residue with less than 6 carbon atoms. Such acrylic co-polymers may have a structure as in Formula 1 in which x represents the fraction of the acrylic acid monomer residues and y represents the fraction of acrylic acid ester monomer residues. The different monomer residues may be randomly distributed along the polymer chain or not.

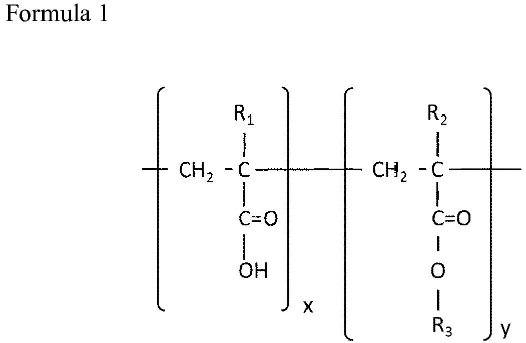

The sum of fraction x and y together may be less than 0.9, or less than 0.8 so that there may be other monomer residues, for example for crosslinking purposes. If there are no other monomer residues present the sum of fraction x and y is 1. A fraction of the carboxylic acid groups may be reacted with pentaerytritol or other di, tri, or tetra alcohol for crosslinking of polymer chains. In such case the sum of x and y may be still 1 while having crosslinked polymers.

The fractions x and y can be chosen to tailor thickening properties of the polymer. Thus x and y may be equally large. Alternatively x may be smaller than y or vice versa, where up to some maximum value, a higher x may increase the solubility upon increasing pH.

A part, or all, of each of the acrylic acid and/or the acrylic acid ester monomer residues may be substituted with Fluorine and/or alkyl groups at their carbonyl α-carbon atoms. Thus, R₁ and R₂ may each be individually chosen to be H, F, or linear or branched alkyl or alkoxy groups of less than 6 carbon atoms, preferably less than 4 carbon atoms. The alkyl or alkoxy groups can have one or more ether groups therein. Ether groups are generally considered as apolar. Preferably, the alkyl groups are methyl and/or ethyl and/or the alkoxy groups are methoxy and/or ethoxy groups. Most preferably the R₁ and R₂ are methyl and/or ethyl groups.

Thus, for example, a fraction x of acrylic acid monomer residues may comprise a first sub-fraction of monomer residues with Ri=H and a second sub-fraction of monomer residues with Ri=methyl. Likewise, a fraction y of acrylic acid ester monomer residues may comprise a first sub-fraction of residues with R₂=H and a second sub-fraction of residues with Ri=methyl.

The alcohol residue R₃ has less than 6 carbon atoms and preferably even less than 5, or 4, or even 3 carbon atoms. R₃ may be aryl, alkyl, cycloalkyl or a combination of these and may comprise ether moieties, but this is not preferred. Preferably R₃ is one or more of methyl, ethyl, propyl, butyl and/or pentyl, linear or branched where possible.

Examples of suitable acrylic ASE co-polymers are the commercially available family of polymers sold under the tradename Carbopol®. Other comparable acrylic co-polymers sold under other tradenames exist. Carbopol® 940 NF polymer may, for instance, be used. The pH of a 0.5 to 1 wt. % Carbopol® 940 NF aqueous solution or dispersion is in the range of 2.5 to 5, with a 1 wt. % solution or dispersion having a pH in the range of 2.5 to 3.0. At such pH, the viscosity of a 1 wt. % polymer solution/dispersion is well below 1500 mPa·s. Already for the 0.5 wt. % polymer variant the viscosity increases to 40,000 - 60,000 mPa·s at a pH in the range of 7.5 to 10.0. At pH of around 7, a 1 wt. % gum-like solution of Carbopol® 940 NF polymer takes the form of a stiff gel. Such Carbopol® 940 NF and other suitable Carbopol® polymers may, for example, be included in the hypochlorite composition at a content in the range of 0.5 wt.% to 5.0 wt.%, preferably 0.5 to 5.0 wt.% to 2.0 to 5.0 wt.%. The polymer content of the system is thus chosen such that after combination of the components of the system the resulting hypochlorite composition has the desired viscosity while in the second component of the system the polymer does not cause a viscosity for example exceeding the desired maximum component viscosity of 1500 mPa·s, 1000 mPa·s, or even 500 mPa·s.

Other suitable acrylic co-polymers can be used such as for example the commercially available polymers sold under the tradenames ACUSOL™ 810A, ACUSOL™ 830, ACUSOL™ 835, ACUSOL™ 842. And yet other ASE type polymers of other structure and or vendors may also be used.

Additionally or alternatively the systems pH induced thickener can comprise a hydrophobically modified alkali-soluble emulsion (HASE) polymer. A HASE polymer is capable of forming a hydrophobically-modified alkali-soluble emulsion when dispersed in an aqueous composition at a pH below 7. HASE polymers may be preferred over ASE polymers as they may provide higher viscosity gels, often in less time, while providing lower viscosity solutions and/or suspensions (such as with viscosity lower than 100 mPa·s when in acidic environment) at similar concentration levels as ASE polymers. Compared to ASE polymers, HASE polymers in general have additional relatively large hydrophobic groups. Without wanting to be bound by theory, the improved thickening effects compared to ASE polymers are believed to be due to the HASE polymers' capability to not only provide thickening through the non-associative mechanism based on polar interactions similar to those occurring with ASE polymers, but also through an associative mechanism based on apolar interactions of the large hydrophobic groups between themselves and between them and other apolar constituents of a composition they are part of (such as for example solvents, dispersion media, surfactants, dispersants etc.).

One group of HASE polymers comprises the aforementioned acidic groups in the form of for example the carboxylic acid groups. Such polymers may form emulsions at acidic pH below 7 due to their reduced deprotonation and hydrophobic modification. The emulsions may have a relatively low viscosity, often lower than for the ASE polymers described herein above. For example the viscosities may be below 500 mPa·s or even below 30 mPa·s. Adding base to such emulsion results in the aforementioned deprotonation of acidic groups and concomitant increase of solubility of the polymer which is accompanied by a degree of swelling of the polymer far greater 5000 mPa·s. For these polymers, the hydrophobic groups are believed to be freed upon dissolution so that they may participate in forming a hydrophobic group interaction based network due to the intramolecular and intermolecular hydrophobic group interactions between such hydrophobic groups and with other hydrophobic entities/molecules in a composition such as surfactants, particles, emulsion droplets, fragrants, flavorants, dyes etc. As a result of this associative mechanism of thickening, which ASE polymers typically are not capable of having, in general a more viscous gel composition results with HASE polymers than with the ASE polymers.

A group of polymers having acidic groups and that may provide good HASE polymers are acrylic co-polymers comprising or consisting of monomer residues of at least one acrylic acid and at least one acrylic acid ester (acrylate residue) having a hydrophobic alcohol residue with 6 or more carbon atoms. Such acrylic co-polymers may have a structure as in Formula 2 in which x represents the fraction of the acrylic acid monomer residues and y represents the fraction of acrylic acid ester monomer residues as defined for the ASE polymers of Formula 1 herein above and z represent the fraction of acrylic acid ester residues having a hydrophobic alcohol residue with 6 or more carbon atoms. Thus, for a HASE polymer, z may not be zero, while y may be zero. The different monomer residues may be randomly distributed along the polymer chain or not.

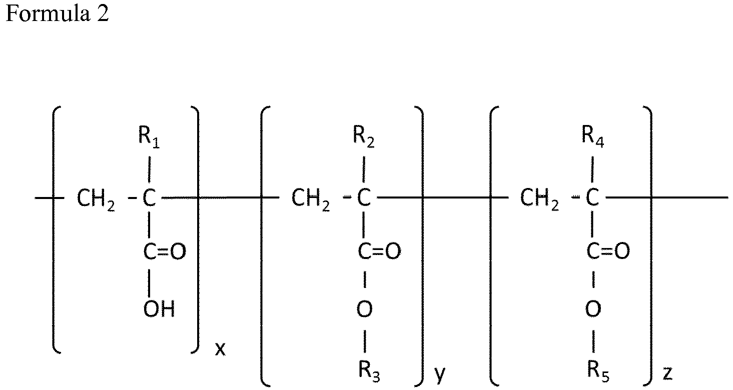

The sum of fraction x, y and z together may be 1 so that there are no other monomer residues present. Alternatively, this sum may be less than 1, such as 0.9, or less than 0.8 so that there may be other monomer residues, for example for crosslinking purposes. A fraction of the carboxylic acid groups may be reacted with pentaerytritol or other di, tri, or tetra alcohol for crosslinking of polymer chains. In such case the sum of x, y and z may be still 1 while having crosslinked polymers.

The fractions x, y and z can be chosen to tailor thickening properties of the polymer. The fractions may all be equally large. Alternatively z may be smaller than x and y or vice versa. Like with the ASE polymers, a higher x in the HASE polymers may increase solubility upon increasing the pH. As indicated herein before, z may not be 0 while y may be 0. Typically however y is also not 0.

Like with the ASE polymers, a part, or all, of each of the acrylic acid and/or the acrylic acid ester monomer residues in Formula 2 may be substituted with Fluorine and/or alkyl groups at their carbonyl α-carbon atoms. Thus, for such monomer residues R₁, R₂ and R₄, may each be individually chosen to be H, F, or linear or branched alkyl or alkoxy groups of less than 6 carbon atoms, preferably less than 5 carbon atoms and more preferably less than 4 carbon atoms. The alkyl or alkoxy groups can have an ether group therein. Preferably, the alkyl groups are methyl and/or ethyl and/or the alkoxy groups are methoxy and/or ethoxy groups. Most preferably the R₁, R₂ and R₄ are chosen from methyl and/or ethyl groups. Thus, in an example, the monomer residue fraction x, and one or more of the fractions y and z, each may comprise a first sub-fraction of monomer residues with Ri=H and a second sub-fraction of monomer residues with Ri=methyl.

The alcohol residue R₃ may be chosen as defined for Formula 1.

The alcohol residue R₅ has 6 or more carbon atoms, preferably 10 or more or even 15 or more carbon atoms. R₅ may be a C₁₀-C₄₀, or C₁₅-C₄₀ or C₁₅-C₃₀ or C₂₀-C₄₀ or C₂₀-C₃₀ group of the type linear or branched alkyl, cycloalkyl, aryl or a combination of these. R₅ may comprise ether moieties, but this is not preferred. In an embodiment the R₅ group is an alkyl or cycloalkyl group.

A particularly desired group of HASE polymers has the Formula 3 and defines a sub-groups of polymers of structure of Formula 2. In this case the fraction x of Formula 2 is given by the total of x1 and x2 in Formula 3. Rx groups may be chosen similar to those for Formula 2 with the following specifications. The fraction y is not 0. Part of the monomer residues of fractions y and z have R₄ and R₆ respectively as H.

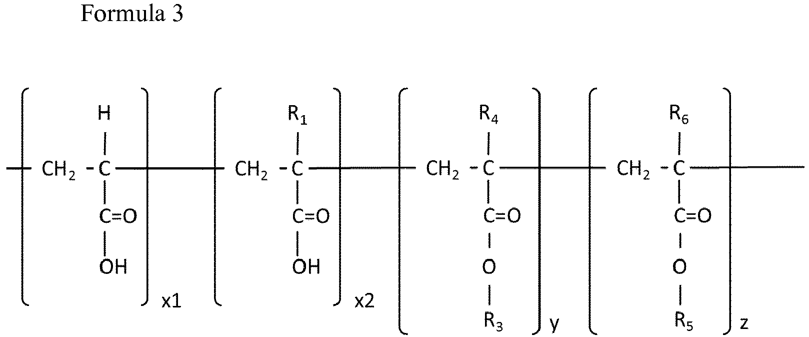

Typically x1, x2, y and z together are all non-zero and their sum is 1, but alternatively their sum may be 0.9 or 0.8 to allow for the presence of other monomers.

Examples of acrylic co-polymer type HASE polymers are commercially available as the polymers sold under the tradenames Acusol™. Others, sold by other vendors exist and can be used as well. The Acusol™ 820 forms an extremely viscous gel with a viscosity > 1.0 x·10⁵ mPa·s at pH in the range of 7.5 to 12.5 at a ∼2 wt. % active polymer content in such gel. By contrast, a very low viscosity of less than 50 mPa·s or even less than 30 mPa·s, may be observed for these polymers at pH 3.0 at contents as high as 30 wt. % in an acidic composition of a system or kit for preparation of such gel. The content of Acusol™ 820, 823 and/or other HASE polymers in the hypochlorite composition may be, for instance, in the range of 0.5 to 20 wt. %. A preferred range is 1 to 10 wt. %, and a more preferred range is 1 to 6 wt. %. Other content ranges of HASE polymers as the ones mentioned above in a hypochlorite composition may, for example, be in the range of 0.5 wt. % to 20.0 wt. %, depending on the viscosity behavior of the polymer. Preferably this content is in the range of 0.5 wt. % to 20.0 wt. % or in the range of 0.5 wt. % to 8.0 wt. %, or between 0.5 wt. % to 5.0 wt. %, or 0.5 to 3.0 wt. %. The lower content boundary in these ranges preferably is 1.0 wt. %.

As an example of how the HASE polymers function, consider combining equal amounts of an aqueous composition with 6.0 wt. % ACUSOL 820 and an aqueous composition of 0.74 wt. % sodium hydroxide, which results in a clear foam-free gel with a viscosity of >100,000 mPa·s while only containing a 0.9 wt. % ACUSOL 820's active polymer solids. Note the dilution factor due to combination of equal amounts of ACUSOL and hydroxide compositions.

All of the pH induced thickeners such as the aforementioned ASE and HASE polymers preferably do not include chemical groups or entities susceptible to oxidation by the hypochlorite within 15 minutes or less. Indeed such is typically the case with the specific groups of ASE and HASE polymers described herein above. In general polymers with saturated bond backbones and acidic groups will have relatively good resistance to the oxidizing agents even at high contents. The HASE type polymers, such as those described herein before have an increased salt or electrolyte content tolerance when compared to the ASE polymers, i.e. their viscosity decrease with increased electrolyte content is less. This is advantageous for high ionic content compositions (hypochlorite being an ionic species in free form) to be used for oral application. After all, the oral application systems may benefit from certain specific additives in salt or electrolyte form such as the remineralization and desensitizing agents as will become apparent hereinafter. HASE polymers are also more compatible with surfactants and fragrances which is useful for oral application systems too.

Another group of pH induced thickener comprises or consists of a hydroxyalkyl cellulose such as for example, hydroxyethyl cellulose or hydroxypropyl cellulose, although these compounds may be less stable against the oxidative hypochlorite, they are uncharged when brought to the composition pH of 7 to 12. This may have advantages compared to the acid group comprising polymers described herein before. As hydroxyethylcellulose powders e.g. Natrosol™ 250 (Ashland®) and CELLOSIZE™ QP 100MH (DOW®) have been found to be effective. These may also be dispersed and/or dissolved in suitable solvents and dispersants such as water alcohol mixtures.

The hypochlorite composition is a thickened or viscous composition. It preferably is a gel such as a hydrogel. The hypochlorite composition preferably has a viscosity that is at least higher than one or more of the kits compositions and most preferably higher than all of the kit compositions (not counting the compositions in dry form). Preferably the hypochlorite composition has a viscosity of at least 1500 mPa·s. More preferably such viscosity is higher than any one of the following values in mPa·s: 2000, 5000, 10,000, 20,000, 50,000, 75,000, 100,000, 200,000, 500,000, 1,000,000.

Upon mixing of the systems components, the thickening may occur at a certain rate of increase of viscosity. For a pH induced thickener at standard conditions, thickening period may be taken as the time period between the moments of mixing of the components of a system and of reaching the minimum viscosity specified for the hypochlorite composition. The shorter the thickening period, the faster the viscosity increase, i.e. higher the rate. The thickening period is, for example, less than any one of the following maximum values in minutes: 5, 2, 1, and 0.5. Preferably it is less than 10 seconds. Preferably such period is achieved for minimum viscosities of 5000 mPa·s, 50,000 mPa·s or even 500,000 mPa·s. The period may be tailored by the type of pH induced thickeners used, HASE polymers in general providing a shorter period than ASE polymers) due to their content (HASE polymers in general providing a shorter period than ASE polymers at same or even lower content) and the pH change invoked. For example, as described herein before, the HASE type polymers may thicken faster than ASE type polymers under similar pH conditions.

The hypochlorite composition to be obtained with the system and method has a hypochlorite composition pH in the range of 7 to 12. When the system is intended to be used on a living human or animal subject, such as when it is to be used for bodily disinfection and in particular oral disinfection, a more body-compatible pH in the range of 7 to 9.5, or 7 to 8 or more preferably in the range of 8 to 9 is used. Otherwise, a composition having a pH in the range of 8 to 12, more preferably in the range of 8 to 10 can be used.

A hypochlorite composition pH in such alkaline ranges in general activates the hypochlorite agent at least to some extent as hypochloric acid has a pKa of 7.53. Thus, while the hypochlorite agent is stored in the system in a component at relatively high alkaline pH of 11 to 13.5 driving the reaction of hypochlorite ions with water to form hypochloric acid to the hypochlorite side, in the hypochlorite composition the lower pH or 7 to 12 allows release of the hypochloric acid.

At the same time, the alkaline pH in the hypochlorite composition directly promotes increase of its viscosity to higher values due to the presence of the pH induced thickener.

The system comprises at least two components. It preferably comprises only two components or only three components of which certain quantities are or that are entirely mixed to arrive at a hypochlorite composition. Furthermore, preferably all of the components comprise a solvent and/or dispersion medium. More preferably such medium includes or consists of water. It may include other solvents and/or dispersion media as described hereinbefore.

In case the components each comprise a solvent and/or dispersion medium, there may be preferred mixing ratios of quantities of the respective components. Such ratios define dilution factors for ingredients of the respective components as upon preparation of a hypochlorite composition mixing of components occurs.

Dilution factors in the range of 1 to 10 may be used. Useful dilution factors are any one of the following: 10, 9, 8, 7, 6, 5, 4, 3, 2, 1.

A suitable first ratio of first quantity of first component to the second quantity of second component in such case is in the range of 10 to 0.1, or 10 to 1. More preferably it is in a range of 5 to 0.2 or even 5 to 0.8. For example a ratio may be chosen from the group of ratios consisting of 10, 9, 8, 7, 6, 5, 4, 3, 2, 1, 0.9, 0.8, 0.7, 0.6, 0.5, 0.4, 0.3, 0.2, 0.1. Such ratios may for example conveniently be employed for two-component systems. However, they may also be chosen for a three component system. For a three component system having a third quantity of a third component, there may be defined second ratio of first quantity to third quantity. Its values may be defined as those of the first ratio and may the same or different. Thus for example, the second ratio may also be in the range of 10 to 0.1 or 10 to 1, etc. Preferably the ratios are the same, such as both 1.

The first and/or second ratios may be based on quantities measured in weights or volume. When contents of compositions and components are measured in weight percentages to the weight of such compositions or components, the ratios may be defined based on the quantities measured in weight. Alternatively, volume percentages can be used in combination with ratios defined in volume percentages. This may be advantageous when all components have a solvent and/or dispersion medium.

Preferably the ratios are based on weight defined quantities of components.

It will be clear to those skilled in the art that the mixing ratios chosen will to some agree be dependent on the nature an amounts of the ingredients present in the components, the desired operative consideration being that the final hypochlorite composition obtained after mixing of the components should have a pH in the desired range, a viscosity in the desired range and a hypochlorite content or other ingredient in the desired range, which ranges have been described herein for their various purposes.

The hypochlorite content in the first quantity of the first composition is preferably chosen such that the content of hypochlorite in the hypochlorite composition is within anyone of the ranges described for the hypochlorite composition, taking into account any dilution upon combination of components and ingredients of a system to arrive at the hypochlorite composition.

Starting from desired content ranges in the hypochlorite composition, useful ranges for total hypochlorite (free and bound form including in hypochlorite sources) in the components may be derived using such mixing ratios and/or dilution factors.

This total content of hypochlorite in the first quantity of the first component preferably is lower than 6.70^{∗}10⁻³ M_{hg}/g (50.0 wt. % NaOCl), more preferably lower than 5.36^{∗}10⁻³ M_{hg}/g (40.0 wt. % NaOCl) and even more preferably lower than 3.35^{∗}10⁻³ M_{hg}/g (25.0 wt. % NaOCl). Lower content is safer. A suitable range is from 1.34^{∗}10⁻⁴ to 2.68^{∗}10⁻³ M_{hg}/g (1.0 to 20.0 wt. % NaOCl), preferably from 1.34^{∗}10⁻⁴ to 2.01^{∗}10⁻³ M_{hg}/g (1.0 to 15.0 wt. % NaOCl), or more preferably from 1.34^{∗}10⁻⁴ to 4.02^{∗}10⁻³ M_{hg}/g (3.0 to 12.5 wt. % NaOCl). Contents are in moles of hypochlorite per gram of first quantity of the first component with between parentheses the equivalent sodium hypochlorite content in weight percentages to the weight of the first quantity of the first component.

The first pH of the first quantity of the first component is in the range of 10 to 13.5, preferably in the range of 11 to 13.5 and even more preferably in the range of 12 to 13. At such pH the hypochlorite is most stable and this provides increased shelf life of the system.

The first quantity of the first component may comprise an excess base to provide these first pH values. Such base may be any base suitable for this purpose and preferably it is soluble in water. Particular suitable bases are hydroxides of ammonium cations or of the alkali metals or alkali earth metals. Carbonate and phosphate salts may also be used. Sodium hydroxide and potassium hydroxide are preferred. Yet other suitable bases may be used. For oral applications of the system the base used should be orally acceptable. An amount of caustic soda in 0.2 to 1.0 % excess to a 3 to 8 wt. % sodium hypochlorite content in the first component will generally provide a first pH in the range of 12 to 13.

The excess base in the first quantity of first component may need to be compensated to reach a desired pH in the hypochlorite composition. Thus, this excess base, together with the total acidic compound content in the second quantity of second component and the optional pH adjusting agent and any optional other acidic or alkaline ingredients will ultimately determine the quantity of second component to be mixed with the first quantity of first component, the goal being a final hypochlorite composition with composition pH in the range specified.

The total amount of pH induced thickener present in all quantities of components of a system to be mixed to arrive at a hypochlorite composition is the same as the amount of pH induced thickener in this hypochlorite composition. Suitable ranges have been given herein before. The hypochlorite content in the first quantity of the first composition is preferably chosen such that the content of hypochlorite in the hypochlorite composition is within anyone of the ranges described for the hypochlorite composition, taking into account any dilution upon combination of components and ingredients of a system to arrive at the hypochlorite composition.

Starting from desired content ranges in the hypochlorite composition, useful ranges for total hypochlorite (free and bonded form including in hypochlorite sources) in the components may be derived using such mixing ratios and/or dilution factors. The amount of pH induced thickener is preferably in the range of 1 to 50 wt. %, more preferably in the range of 1 to 30 wt. % and most preferably in the range of 5 to 25 wt. %. These ranges may combine well with the suitable dilution factors and mixing ratios provided herein above.

The second quantity of the second component has a pH of less than 7 when it includes a solvent and/or dispersion medium such as one including water. Such second pH reduces, minimizes or prevents the pH induced thickener to cause substantial thickening of second quantity. Preferably the second pH is less than 6, less than 5 or even less than 4. More preferably the second pH is in the range of 1 to 6, or 2 to 6, or 2 to 5 or 2 to 4. For example the acrylic copolymers disclosed herein, including such ASE and HASE polymers, may then be protonated to large extent so that they are in low viscosity forming form which may be an emulsion or dispersion. The second component can include a pH adjusting agent as described hereinafter to adjust the pH to a required level.

The pH adjusting agent can comprise or consist of one or more acidic compounds. The presence of such pH adjusting agent may be to compensate the excess base of the first quantity of first composition. Alternatively or additionally it may serve to set a required level for the second composition when added to it. The acidic compound may be a strong acid like hydrochloric acid or phosphoric acid and/or may be a weak acid such as for example a carboxylic acid like acetic acid, citric acid or the like.

In some embodiments of the system, the pH induced thickener is an acidic compound itself. This is for example the case with the acidic group comprising polymers such as some of the ASE and HASE polymers described herein before. In such case the pH adjusting agent may not be needed at all. This may provide simpler system and cheaper to make.

Alternatively the additional pH adjuster may still be used to tune pH values.

The aqueous first composition and/or any further system component having a solvent and/or dispersion medium may have a viscosity that is typically lower than that of the hypochlorite composition. The constituents and their content in a particular system composition are preferably independently chosen so that a composition has a viscosity below 1500 mPa·s, more preferably below 1000 mPa·s or most preferably below 500 mPa·s. In an optimum situation such viscosity is lower than 100 mPa·s. Preferably, all of the system compositions have such viscosity. Viscosity values below 500 mPa·s and in particular below 100 mPa·s provide good rheological flowability and therewith easier handling of components during manufacture and use. In some embodiments, for example the viscosity of an aqueous second composition of pH lower than 7 including an acrylic co-polymer HASE type pH induced thickener can have a viscosity of less than 50 mPa·s or lower.

The hypochlorite composition and the system preferably is one for oral application in that it can be used for prevention or treatment of oral infection. In such case the hypochlorite composition and system may include one or more additional agents in the form of a tooth desensitizing agent, a tooth remineralizing agent, a coloring agent and a flavoring agent.

Any one or more of these additional agents may be added to one or more of the components of the system provided that such additional agent is to at least some extent chemically compatible with chemical conditions (e.g. pH or oxidizing) within such components. It is noted here that while the first component is alkaline and oxidizing, the second component is acidic and thus, when chemical stability of such additional agent is reduced within a particular component, the additional agent may be omitted from that particular component and only added to the other. If it is not compatible to conditions of either of the components they may be added to the system in the form of a third component. Such third component may for example have a neutral pH and/or may lack any oxidizing species.

An amount of additional agent in a system may be entirely present in one component of the system, but may also be distributed over multiple components. This may help to control concentrations of such additives in components.

In some embodiments precursors of additives are distributed over multiple components. For example, amorphous calcium phosphate (ACP) are preferably prepared in situ from more stable precursors distributed over the different components of the system as well be described hereinafter. In another example additives are salts that may be dissolved and dissociated in ions. The cations and anions may be provided as different precursors in different components as will be described hereinafter.

In an embodiment the desensitizing agent comprises or consist of potassium ions or one or more sources of potassium ions such as potassium salts. The potassium ions are believed to desensitize nerve fibers blocking the neural transmission. The one or more potassium salts may be chosen from the group consisting of: potassium chloride, potassium fluoride, potassium citrate, potassium nitrate, potassium carbonate and potassium hydroxide. Other potassium salts can be used. The desensitizing agent or parts of it may be present in two or more components. Thus, the first component can have for example potassium hydroxide while the second component includes potassium chloride or potassium nitrate. Alternatively, the potassium ions can be part of the first component e.g. as potassium hydroxide, while counterions such as chloride and nitrate ions are part of the second component with cations different from potassium.

The content of potassium ions and counter ions should be controlled in their related compositions so that they will produce no more potassium than the equivalent of 5 wt. % potassium nitrate in the hypochlorite composition to meet regulations requirements. The preferred range of potassium ions in the components of a system amounts to the equivalent present in 1.0 wt. % - 4.5 wt. % of potassium nitrate in the hypochlorite composition. Again dilution factors and mixing ratios as described herein before may need to be taken into account.

In an embodiment the desensitizing agent or remineralization agent comprises or consists of one or more compounds also suitable for plugging dental tubules. For example, such compounds may be any one of: Sodium fluoride, Stannous fluoride, Strontium chloride, Silver diamine fluoride, Potassium oxalate, Calcium phosphate, Calcium carbonate. Preferably the system comprises a calcium ions or a source of calcium ions and/or strontium ions and monobasic, and/or dibasic and/or tribasic phosphoric acid ions (phosphoric ions) or a source of any one or more of these. Preferably the calcium and/or strontium ions or their sources are comprised within an acidic component such as the second component e.g. in soluble salt form such as e.g. calcium nitrate and/or strontium nitrate. Such compounds need the acidic environment if they are required to be in solution. The phosphoric ions and their sources are preferably comprised within an alkaline environment such as in the first component. The separated sources (precursors for the desensitizer and/or remineralizing agent) system allows convenient storage, while it provides in situ formation of calcium phosphate (CP) and/or strontium phosphate (SP) in crystalline or, more preferably amorphous form (ACP and ASP) in the hypochlorite composition upon mixing of the components when the system is used.

Generally, the content of calcium ions and the content of phosphoric ions or carbonate ions in the system compositions should be dosed within the range that can produce 0.1 wt. % to 1.0 wt. % CP or ACP, respectively, (around 0.5 wt. % preferred) in the finished hypochlorite composition.

Detailed materials and contents of additives such as ACP or potassium nitrate that can be used in the current systems and how they are to be distributed over multiple compositions of a system are disclosed in US9138386B2, which is incorporated by reference in its entirety.

In an embodiment the desensitizing agent comprises or consists of the one or more compounds for plugging dental tubules and potassium ions and/or a source of potassium ions.

Suitable surfactants for the hypochlorite composition or any one of the system components include polyol surfactants, such as Pluronic F-127. Other surfactants, such as Cremophor RH-40, USP and Crodateric CAB 30-LQ, may also be employed.

The hypochlorite composition and the system may further comprise a flavoring agent. The flavoring agent may be present in one or more components of the system. The flavoring agent may, for instance, be included in the aqueous first component and composition, and/or the second composition. Alternatively it may be present in an optional third component and third composition. The possibility of including the flavoring agent in the composition separate from the first composition may enable more flexibility in terms of the flavor options because providing the flavoring agent separately in this manner reduces the risk that the flavoring agent is degraded by a strong oxidizing agent. This contrasts with conventional systems which tend to have a restricted flavor choice because of the requirement to select flavoring agents according to their stability over a relatively long storage time in the presence of the other ingredients.

A suitable flavoring agent may include natural peppermint oil, which may provide the user with a sense of cleanliness and freshness, particularly when the hypochlorite composition is applied in treatment of oral disease which may be accompanied by the bad breath. In some non-limiting examples, sweeteners, such as xylitol, sodium saccharin, may also be used, albeit usually more sparingly as compared with other sweetened products, since excessive sweetness may be undesirable for oral products, due to the negative perception of sweetness in relation to dental health.

**Table 1**

| Two-component system | |
|---|---|
| first component | • Solution or dispersion of an amount of hypochlorite salt (sodium or potassium hypochlorite) in a range of 1 to 40 wt. %, preferably 3 to 20 wt.% to the weight of the first component; |
| | • first pH in the range of 10 to 13.5 and preferably in the range of 12 to 13; |
| | Optionally: |
| | • At least one additive of: K⁺ ions (e.g. from KOH or KNO₃) and phosphate ions (e.g. from Na₂HPO₄) |
| second component | • Solution or dispersion of amount of pH induced thickener in the form of ASE and/or HASE polymer of acrylic copolymer type as disclosed herein), in range of 1 to 25 wt. % to the weight of the second component. |
| | • Second pH below 7 preferably in the range of 2 to 5. |
| | Optionally: |
| | • pH adjusting agent to tune pH. |
| | • At least one additive of: Ca²⁺ and/or Sr²⁺ ions (e.g. from CaCl₂); K⁺ ions (e.g. from KNO₃) and Flavoring agent(s); solvent (e.g. ethanol and/or polyethylene glycol); surfactant(s); Flavoring agent(s). |

Example systems having components with preferred ranges of constituents and parameters are provided in Tables 1 and 2. These systems can be used for disinfection and sanitization purposes. The final concentration of hypochlorite may determine applicability to oral.

**Table 3**

| Three-component system | |
|---|---|
| First component | • Solution or dispersion of an amount of hypochlorite salt (sodium or potassium hypochlorite) in a range of 1 to 40 wt. %, preferably 3 to 20 wt.% to the weight of the first component; |
| | • first pH in the range of 10 to 13.5 and preferably in the range of 12 to 13; |
| | Optionally: |
| | • at least one additive of: K⁺ ions (e.g. from KOH or KNO₃); phosphate ions (e.g. from Na₂HPO₄) |
| Second component | • Solution or dispersion of amount of pH induced thickener in the form of ASE and/or HASE polymer of acrylic copolymer type as disclosed herein), in range of 1 to 25 wt. % to the weight of the second component; |
| | • Second pH below 7 preferably in the range of 2 to 5; |
| | Optionally: |
| | • pH adjusting agent to tune second pH; |
| | • at least one additive of: Ca²⁺ and/or Sr²⁺ ions (e.g. from CaCl₂); K⁺ ions (e.g. from KNO₃) and Flavoring agent(s); solvent (e.g. ethanol and/or polyethylene glycol); surfactant(s); Flavoring agent(s). |
| Third component | • pH adjusting agent in addition to or as alternative to pH adjusting agent in second component. |
| | • at least one additive, of: e.g. solvent(s); surfactant(s); K⁺ ions (e.g. from KNO₃) ; source of phosphate ions (e.g. from Na₂HPO₄); Flavoring agent(s); |

The at least one additives may be present in all of the compositions, but may also be present in only one or two of the compositions. For example if an additive is not compatible with certain compositional characteristics such as e.g. alkaline pH or acidic pH it may be added to an appropriate one or not at all. The third composition preferably has pH of 7. It may have only flavouring agents.

In one example there may be more components than three. For example the flavoring agent may be separated from the first, second and third components in a fourth component. This component may have a pH of 7. Alternatively, such fourth component may lack any solvent and/or dispersion medium. If an additive is not compatible with either basic or acidic conditions it may be separated from such basic or acidic compositions in such fourth component.

There may be a trade-off with the number of components in a system and the convenience of preparing the hypochlorite composition with such system. The two component and three component system are most preferred when it comes to convenience during manufacture and use. For example when several separate compartments are required to contain the respective components, the complexity of the design of the dosing system may also increase.

Specific examples of two-component systems, are provided in Table 4. Concentration of an ingredient are in wt. % to the weight of the component they are part of.

By using the mixing ratios of the system components concentrations of ingredients in hypochlorite composition can be calculated. For the two component systems of Table 4, both components are aqueous compositions as they comprise water. Therefore their densities are approximately the same and dilution may be based on volume ratios mixed to arrive at concentrations in a hypochlorite composition.

**Table 4**

| **First component** | | | |
|---|---|---|---|
| **System 1** | | **System 2** | |
| Ingredient | wt.% | Ingredient | wt.% |
| H₂O | 95.94 | H₂O | 42.86 |
| 5.25 wt. % NaOCl + 0.35 wt.% NaOH in water | 4.00 | 5.25 wt. % NaOCl + 0.35 wt.% NaOH in water | 57.14 |
| KOH | 0.06 | | |
| | | | |

| **Second component** | | | |
|---|---|---|---|
| **System 1** | | **System 2** | |
| Ingredient | wt.% | Ingredient | wt.% |
| H₂O | 60.22 | H₂O | 73.72 |
| Citric acid | 0.08 | Citric acid | 0.28 |
| Acusol™ 820 | 6.00 | Acusol™ 820 | 8.00 |
| Propylene Glycol | 16.00 | Propylene Glycol | 4.00 |
| Glycerine | 8.00 | Glycerine | 8.00 |
| Sodium Lauryl Sulfate (30%) | 4.80 | Sodium Lauryl Sulfate (30%) | 6.00 |
| KCl | 4.00 | | |
| Nat. Peppermint Oil PE-5523 | 0.90 | | |

The first and second components of each of the systems 1 and 2 may for example be combined in a volume ratio of first/second = 1 to result in an approximately 0.1 wt. % hypochlorite composition for system 1 and an approximately 1.5 wt. % for system 2. The ratio may be smaller than 1 such as 0.8, 0.6, 0.4 or even 0.5. The hypochlorite agent content in the firsts component of each system may be increased at the expense of water in that component to increase a content of hypochlorite in the final hypochlorite composition.

Furthermore, while the viscosity of the aqueous components of both systems will typically be in the range of only 15 to 20 mPa·s, with such mixing ratio, the hypochlorite compositions formed will be hydrogels with high viscosities of >100,000 mPa·s for system 1 and >200,000 mPa·s for system 2. The formation of such gels will be almost instantaneous.

The first pH of the aqueous first component in the systems 1 or 2 are in the range of 12 to 13.5.

The pH of the aqueous second component in the systems is typically in the range of 2.0 to 4.0 depending on the grade and quality of the Acusol™ 820, which is a pH induced thickener in the form of a HASE acrylic co-polymer. If a change of type or content of such pH induced thickener is desired, in order to change e.g. viscosity requirements, a suitable acid may be used such as for example phosphoric acid to adjust the first pH.

While both may be used for sanitization purposes, the system 1 is suitable for oral application. Other volume or weight ratios of the compositions may be used as indicated herein before to arrive at different content hypochlorite compositions. Thus, a mixing ratio of 2 may result in more concentrated hypochlorite compositions. It will be clear that in such case a final pH and/or viscosity may be slightly different due to different concentrations of the acidic, basic ingredients, and pH induced thickener in the hypochlorite composition.

An example of a 3-component system 3 is provided in Table 5. Concentration of an ingredient are in wt. % to the weight of the component they are part of.

**Table 5**

| **First component** | |
|---|---|
| Ingredient | wt. % |
| H₂O | 64.00 |
| 5.25 wt. % NaOCl + 0.35 wt.% NaOH in water | 36.00 |
| | |

| **Second component** | |
|---|---|
| Ingredient | wt. % |
| H₂O | 87.91 |
| Citric acid | 0.09 |
| Acusol™ 820 | 12.00 |
| | |

| **Third component** | |
|---|---|
| Ingredient | wt. % |
| H₂O | 70.00 |
| Propylene Glycol | 8.00 |
| Glycerine | 16.00 |
| Sodium Lauryl Sulfate (30%) | 6.00 |

The volume ratio used to mix the three components is for example, 1 to 1 to 1 to afford a final composition with 1.5 wt. % hypochlorite. Using the mixing ratios of the components allows calculation of concentrations of ingredients in the final hypochlorite composition. While the viscosity of the aqueous compositions of system 3 will typically be in the range of only 20 to 40 mPa·s, with such mixing ratio, the hypochlorite composition obtained with a mixing ratio of 1 to 1 to 1 will be a hydrogel with high viscosities of >1,000,000 mPa·s. The formation of such gels will be almost instantaneous.

Again the first pH of the aqueous first component in the system 3 is in the range of 12 to 13.5. The pH of the aqueous second component is typically in the range of 2.0 to 4.0 depending on the grade and quality of the Acusol™ 820. If a change of type or content of such pH induced thickener is desired, in order to change e.g. viscosity requirements, a suitable acid may be used such as for example phosphoric acid to adjust the first pH. The third pH of the third composition in system 3 is around 7 (neutral).

In all of the examples, the 5.25 wt. % hypochlorite with 0.35% Excess Caustic Soda solution added to a system component is a commercial household type hypochlorite product. Such products can be purchased with a hypochlorite content of between around 3 and 8 wt. % and contains an excess of base to stabilize the hypochlorite. Any of such solutions can be used but the quantities of ingredients needs to be adjusted to some extent to account for the different concentrations. Sodium hypochlorite may be replaced by other hypochlorites as indicated herein before.

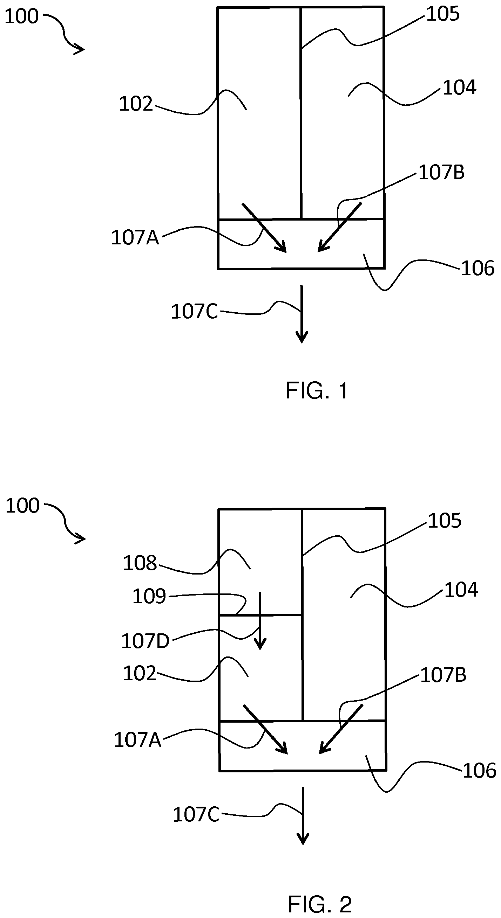

FIG. 1 depicts a kit or system 100 in which the first component 104 is separated from the second component 102 by a dividing wall 105 which ensures that the first component 102 does not contact or combine with the second component 102 prior to combining of the respective component 102, 104.

Separation of the first component 104 and the second component 102 may be implemented in any suitable manner. For example there may be multiple vessels, bottles or syringes or multiple compartments of a single device. The device may have a means for mixing such as mixing vessel and/or spatula.

The single multi-compartment device can for example comprise a multi-compartment bottle, multi-compartment syringe, or an alternative multi-compartment dispensing system capable of separately containing the components of the kit or system. The compartments may be made of any material suitable for storing the components and ingredients. Particularly suitable are materials substantially resistant to the hypochlorite agents and/or acidic and/or basic conditions. Such materials may be plastics such as e.g. polyethylene, polypropylene, or other or may be glass.

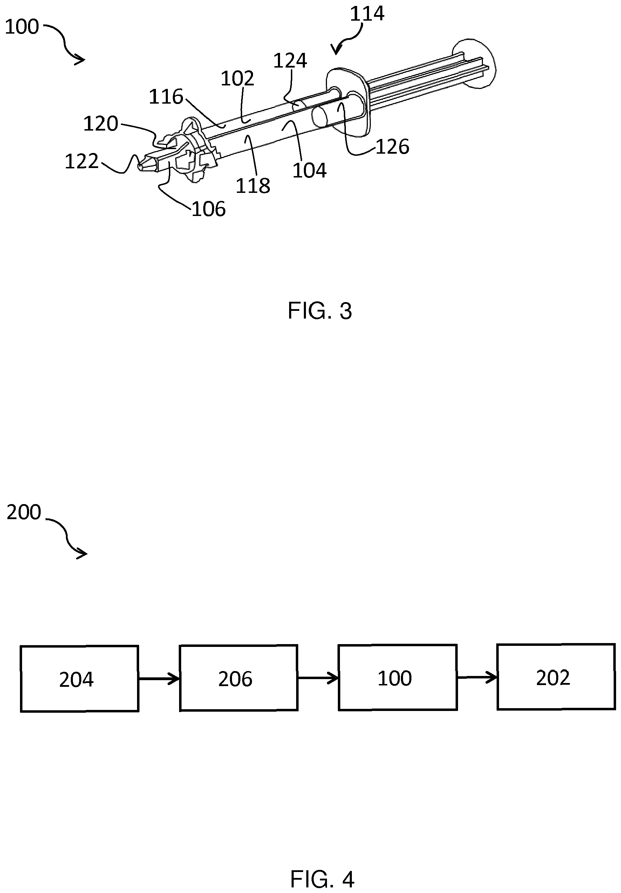

The kit or system 100 comprises (and this is optional) mixing chamber 106 (may be a simple container or compartment) in which the respective components 102, 104 may be combined or mixed. The arrow 107B represents the passage of the first component 104 into the mixing chamber 106. The arrow 107A represents the passage of the second component 102 into the mixing chamber 106. Upon mixing of the respective components in the mixing chamber 106, the alkaline pH triggers the pH induced thickener to cause the increase of viscosity. The resulting mixture may then be retrieved from the chamber and used. The retrieval or passage of the hypochlorite composition out of the container 106 is schematically represented by the arrow 107C. The mixing chamber 106 may, for instance, be included as or in a mixing tip of a syringe, as will be explained in more detail with reference to FIG. 3. The retrieval of composition form the mixing chamber may be done directly upon mixing so that full viscosity increase may not yet have been achieved. This may be advantageous as many of the kit embodiments will eventually result in high viscosity hypochlorite compositions that sometimes may be difficult to manipulate or retrieve from the chamber. For example if it needs to flow through narrow openings or chamber outlet or the like. Alternatively, there may be a waiting time to have the mixture achieve its final viscosity. Manipulation using a spatula may then be easier.

The pH induced thickener is included in the second component in the kit or system. As described herein before, this may simplify the kit or system 100 relative to the scenario where the second component 102 and the pH induced thickener are provided separately from each other.

FIG. 2 shows an example in which the pH induced thickener is provided separately from the other ingredients of the second component 102. In this case, the pH induced thickener is provided in a third component 108 separated from the second component 102 by the further dividing wall 109. Both the pH induced thickener and the first component 104 are separated from the second component 102 by the dividing wall 105.

Alternatively, the first component 104, the second component 102 and the third component 108 may, for example be contained in respective compartments of a tri-compartment bottle, a tri-compartment syringe, or an alternative tri-compartment dispensing system capable of separately containing the respective compositions 102, 104, 108. For example in the tri-compartment syringe there would be three compartments each having an outlet towards the mixing chamber 106 such that upon use the contents of these compartments is mixed together simultaneously.

In the example of FIG. 2, the second and third components are mixed together before or during mixing of the resulting intermediate mixture with the first composition. Initial forming of a pre-combination of the second component 104 and the pH induced thickener under acidic conditions may permit its effective, e.g. intimate, mixing with the hypochlorite agent, and any other ingredients contained in the second component 102, at still low viscosity. Subsequent mixing of the pre-combination with the first component 104 may afford the higher viscosity hypochlorite composition, much like described for the kit or system of FIG. 1.

This pre-combination is represented in FIG. 2 by the arrow 107D between the third component 108 and the second component 102. The pre-combination may be carried out in any suitable manner, such as by manually dispensing the pH induced thickener into the second component 102, or vice versa. More elaborate means of carrying out the precombining may also be contemplated, such as by providing a frangible further dividing wall 109; the user breaching the further dividing wall 109 so as to permit the second component 102 and the third component 108 to combine with each other.

The pre-combination composition is supplied to the mixing chamber 106, as schematically represented by the arrow 107A. The first component 104 is also supplied to the mixing chamber 106, as represented by arrow 107B. Following combining, e.g. mixing, of the pre-combination composition and the first component 104, the resulting hypochlorite composition may be retrieved from the mixing chamber much like descried for the example of Fig. 1. Again, the mixing chamber 106 may, for instance, be included as or in a mixing tip of a syringe, as will be explained in more detail with reference to FIG. 3.

The kit or system 100 of FIG. 3 comprises a dual-compartment syringe 114. A first compartment 116 of the syringe 114 contains a first component 104. A second compartment 118 of the syringe 114 contains a second component 102. Whilst a dual-barrel syringe 114 is shown in FIG. 3, a third barrel (not shown) may also be included, e.g. for the third composition 108, as previously described, but this is not shown for reasons of brevity. From the description of the kit or system and syringe of Figs. 2 and 3, those skilled in the art will know how to implement a triple-barrel syringe having the desired contents.

In a kit or system of Fig. 3 as described herein before, the third component 108 preferably is a composition having a solvent and/or dispersion medium of a pH less than 7.

In an alternative example, the third composition 108 may be solvent-free and/or dispersion medium free; the pH induced thickener for example being included in a powder form.

In preferred examples of any of the kits or systems of FIGs 1, 2 or 3, the first component and/or the second component include a solvent and/or dispersion medium. Preferably this includes water.

Any of the kit or systems described herein before can be used with the multi-compartment devices described herein when taking account of the number of separate components and necessary compartments.

The syringe 114 includes a mixing tip 120 for receiving the contents of the respective barrels 116, 118, and that of a further barrel in case a third component is present. This does not mean that the mixing tip must be able to hold all of the contents together, but merely that it must be capable of receiving portions of the respective compartment contents at a particular time. In this respect, the mixing tip 120 includes the mixing chamber 106. Moreover, an outlet 122 is provided in the mixing tip 120 for permitting the hypochlorite composition to exit the syringe 114.

Such a multi-barrel syringe 114 may provide a convenient means of providing the kit 100 to the user. The respective compositions 102, 104, and optionally 108 may be combined in the mixing tip 120, i.e. upon supplying the mixing tip 120 with the respective compositions 102, 104, 108. Plungers 124, 126 may, for instance, be used to force the compositions out of the respective barrels and into the mixing tip 120. Continued forcing, e.g. via the plungers 124, 126, may cause the composition to emerge from the outlet 122, whereupon the composition may be used.

A detailed description of how such syringe, and/or an appropriate mixing tip may be designed and used is provided for example in US8581998 and US6698622 which are incorporated by reference.

Numerous alternative means of implementing the kit or system 100 may also be contemplated, such as using a multi-chamber, e.g. dual-chamber, bottle, or a dispensing system having a plurality of compartments. For example, the system may include two compartments which act as respective reservoirs for the kit or system components; the reservoirs being filled according to the required mixing ratio of the respective components 102, 104.

FIG. 4 shows a block diagram of a system 200 for dispensing a hypochlorite composition. In the dispensing systems 200 the compartments may be discrete and separable (such as with separate bottles) from each other, or may be integrated as three reservoirs in the same device. The system may, for instance, comprise a suitable pump or vacuum arrangement for effecting simultaneous withdrawal of component from each compartment, via separate draw pipes, and for subsequently discharging the respective components into a mixing chamber 106 that may be part of or separate from the system. The resulting composition just after mixing may be expelled or retrieved from the mixing chamber as described for the example of Fig. 1. The system 200 may comprise a kit or system 100 as described herein before and a dispensing pump 202 for dispensing the hypochlorite composition from the mixing chamber 106.

The kit 100 may, for example, take the form of a replaceable cartridge for the system 200, with the respective components 102, 104, and optionally 108 and separate parts, if present, being contained in separate compartments of the cartridge.

As shown in the block diagram of FIG. 4, the system 200 comprises a user interface 204 for enabling user selection of parameters relating to the final hypochlorite composition to be obtained from the system. Parameters may alternatively or additionally be selected via the user interface 204 in respect of the first separate part and/or the second separate part. The parameters may, for example, relate to which of the various components 102, 104, 108 and parts to include in the composition, and/or their respective quantities.

The system 200 shown in FIG. 4 includes a selection module 206 coupled to the user interface 204. The selection module 206 controls dosing of the respective components to the container/mixing chamber 106 based on the user selection provided by the user through the user interface. The selection module 206 may, for example, enable simultaneous withdrawal of the components 102, 104, 108 from their respective compartments, via separate draw pipes, and discharge the fluid to the container/mixing chamber 106.

The user interface 204 may, for instance, enable the user to select parameters which dictate the nature of the composition ultimately dispensed by the system 200. For example, the consistency of the composition may be controlled by the pH induced thickener included in the composition and/or the pH of the composition. An oral application may require less basic conditions than a non-oral application. According to the user selections, the selection module 206 may provide an accurate and efficient means of dispensing the respective component 102, 104, 108 and parts which constitute the composition.

In particular, the parameters may include selection of a flavoring agent or flavoring agents. As previously noted, the possibility of including the flavoring agent in, for instance, the second separate part may enable more flexibility in terms of the flavor options because providing the flavoring agent separately in this manner reduces the risk that the flavoring agent is degraded by other ingredient(s) of the kit 100, such as the hypochlorite compound over a relatively long storage period/shelf life. The system 200 may therefore permit the user to select the flavor(s) of the composition dispensed by the system 200 according to personal preference, in a manner similar to soft drink flavor selection using a soda fountain.

In a simple example, the user may select either to include the flavoring agent in the composition or not to include the flavoring agent in the composition. This requires the flavoring agent to be separate from any of the other components. In a more elaborate example, the user may select from different flavoring agents included in the kit 100. The user interface 204 may, for instance, take the form of a touchscreen to enable user-friendly parameter selection. Since flavor and/or smell may play an important role in consumer product preference, touchscreen options of hypochlorite based gel vending machine may be designed as flavor or fragrance choices.

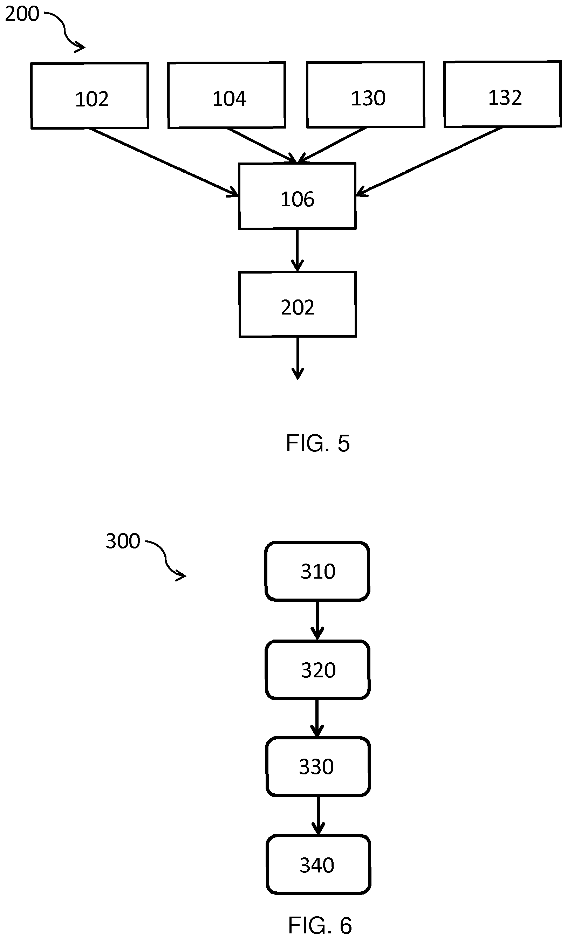

FIG. 5 shows a block diagram of a system 200 according to an embodiment. In this non-limiting example, the system 200 includes the first component 104 and the second component 102 as well as optional further components 130 and 132. The dispensing pump 202 dispenses the hypochlorite composition from the mixing chamber 106.

Any suitable mixing ratio (e.g. for the first component 104, the second component 102) may be employed, according to the desired parameters defined by the user (e.g. hypochlorite content, pH and the content of any other functional ingredients which are to be included in the composition) as long as the required composition pH is within the required range using a particular set of components.

The mixing ratio of each of the compositions of the kit or system may be determined by the desired characteristics of the hypochlorite composition, in relation to its intended purpose, e.g. tooth whitening or sanitization. This system 200 may accurately control dosage of each of the respective components 102, 104, and optional ones 108, 130, 132. Each of these components may be, for instance, supplied in cartridge form to facilitate dosing control and replacement of the components in the system 200. Such a replaceable cartridge-based system 200 may further facilitate the flavoring agent selection described above.

The kit or system examples and the described devices and systems are preferably designed to accommodate any of the previously described system components and to define their mixtures such as to output a desired hypochlorite composition.

FIG. 6 shows a flowchart of a method 300 according to an embodiment. The method 300 is for preparing a hypochlorite composition having a pH in the range of 7 to 12. In step 310, a first composition comprising a hypochlorite agent is provided e.g bought from a vendor or custom made. In this case it is an aqueous component as it includes water.

A pH induced thickener as described herein before is provided in step 320. It may be obtained from a vendor or be custom made. The pH induced thickener maybe dissolved and/or dispersed in a solvent or dispersion medium at the first pH and/or be in dry form. Many of the polymers may be obtained as acidic emulsions from different vendors and these may be used as is when the pH has a value in line with the second pH.

In an optional step 330, a third component comprising a flavoring agent and/or a pH adjuster is provided e.g. from a vendor or custom made.

In step 340, the first component and the second component, together with the optional third component, are combined preferably mixed to form the hypochlorite composition having the alkaline pH in the range of 7 to 12. Accordingly the hypochlorite composition thickens (e.g. because it forms a gel) to provide the advantages described herein before.

The combination step 340 may comprise forming a pre-combination of the second component and the pH induced thickener. The pre-combination may then be combined with the first component, thereby to afford the hypochlorite composition. As explained herein before this may be beneficial as it permits mixing at still low viscosity advantageous for a uniform mixing result.

The method, the hypochlorite composition and therewith the kit and system therefore may be formulated to be suitable for sanitization in general or for disinfection of the human body and in particular the oral cavity. It may be made for retail, take-home or chairside-type application in the oral healthcare sectors. The composition and thus the kit and system therefore may also be formulated for use in sanitization applications, e.g. when relatively long sanitizing times during which the gel-like composition is applied to surfaces are required. The composition and kit and system therefore may be employed for sanitizing surfaces (including human body surfaces, such as the hand, foot, etc., when applicable) at, for instance, the dental office, hospital, nursing home, sports venue, food-processing plant, etc. The kit 100 and system may be designed, for instance, into a vending machine-like system 200, as described above, which may be placed at those facilities.

In summary, described is a multi-component system, and a method for preparing a hypochlorite composition with increased viscosity with a composition pH in the range of 7 to 12. For example, and preferably, the system and method may be used to provide (hydro)gelled hypochlorite composition. The system or method make use of a first quantity of a first component including hypochlorite or a source of hypochlorite at a pH in the range of 10 to 13.5 preferably 11 to 13.5 and a separate component including a pH induced thickening agent in "dry" form or in dissolved and/or dispersed form at a pH less than 7. The pH induced thickener is capable of increasing the viscosity of a composition upon subjecting it to an increasing pH such as in particular the composition pH in the range of 7 to 12. The pH induced thickener is thus for causing an increase of viscosity in the hypochlorite composition caused by the alkaline composition pH experienced by the pH induced thickener. A pH adjusting (acidic or basic) agent may be present to adjust the composition pH to the desired range. The hypochlorite composition is obtainable by combining amounts of the components in the system.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A multi-component system (100) for preparing a hypochlorite composition having a composition pH in the range of 7 to 12, the system comprising:
- a first quantity of a first component comprising hypochlorite and water and having a first pH in the range of 11 to 13.5;
- a second quantity of a second component separate from the first component, the second quantity comprising an amount of pH induced thickener capable of thickening a composition including water upon an increase of the pH of the composition from an acidic to an alkaline value, the amount of pH induced thickener being present in at least one of:
- a solvent and/or dispersion medium free form; and
- a solvent and/or dispersion medium containing form in which the pH induced thickener is dissolved and/or dispersed at a second pH lower than 7; and
- optionally, an amount of a pH adjusting agent chosen to cause the hypochlorite composition to have the composition pH, the amount of pH adjusting agent being separate from the first composition;
the hypochlorite composition being obtainable by combining at least the first quantity of the first component, the second quantity of the second component and optionally the amount of pH adjusting agent.

2. The system according to claim 1, wherein the pH induced thickener comprises or consists of one or more polymers each comprising monomer residues wherein at least a fraction of the monomer residues comprises acidic groups.

3. The system of claim 2 wherein the acidic groups comprise or consist of one or more groups chosen from carboxylic acid groups, sulfonic acid groups, and phosphoric acid groups.

4. The system according to any one of claims 1 to 3, wherein the pH induced thickener comprises or consists of one or more alkali soluble emulsion (ASE) polymers and/or one or more hydrophobically-modified alkali-soluble emulsion (HASE) polymers.

5. The system according to any of the previous claims wherein the pH induced thickener comprises one or more acrylic co-polymers having the acidic groups at least in the form of carboxylic acid groups.

6. The system (100) according to any one of the claims 1 to 5, wherein the pH induced thickener comprises one or more cellulose polymers having residues at least a fraction of which comprise hydroxyl groups.

7. The system of any one of the previous claims, wherein the pH induced thickener of the second quantity is entirely in the solvent and/or dispersion medium containing form.

8. The system of any one of the previous claims, wherein the pH adjusting agent comprises or consists of the pH induced thickener.

9. The system according to any one of claims 5 to 8, wherein the ratio of the first quantity/the second quantity is in a range of 10 to 0.1, preferably in the range of 5 to 0.2, or most preferably in the range of 5 to 0.8.

10. The system according to any one of the previous claims, wherein the second component comprises a solvent and/or dispersion medium and has a viscosity lower than 1000 mPa·s, preferably lower than 500 mPa·s, most preferably lower than 100 mPa·s at standard conditions.

11. The system according to any of claims 1 to 10, wherein the second quantity of the second component comprises a second amount of the pH induced thickener the second amount being sufficient for causing the hypochlorite composition to have a viscosity higher than 50000 mPa·s, more preferably higher than 100000 mPa·s at standard conditions.

12. The system according to any one of claims 1 to 11, comprising one or more of:
- a tooth desensitizing agent;
- a tooth remineralizing agent; and
- a flavoring agent.

13. The system according to any one of claims 1 to 12, comprising a multi-compartment syringe (114), said syringe comprising:
- a first compartment (116) containing the first quantity (102); and
- a second compartment (118), comprising the second quantity; and
the system further comprising a mixing compartment (120) attachable to or attached to the syringe for receiving and mixing the first quantity and the second quantity and having an outlet for permitting the resulting hypochlorite composition to exit the syringe upon the use of the syringe.

14. The system according to any of the claims 1 to 13, comprising a dispensing system (200) for dispensing the hypochlorite composition, the dispensing system comprising:
- a mixing unit for combining quantities of the first component, the second component and optionally the pH adjusting agent to therewith provide the hypochlorite composition;
- a user interface (204) for enabling a user to provide input parameters relating to at least one of:
- use of the hypochlorite composition;
- a hypochlorite content in the hypochlorite composition;
- the composition pH;
- the viscosity of the hypochlorite composition; and
- flavor and/or smell of the hypochlorite composition;
- a selection module (206) for controlling the mixing unit to determine the quantities of components to be combined for the hypochlorite composition based on the input parameters.

15. The system of any one of claims 1 to 14, for providing a hypochlorite composition for use as an orally applicable disinfectant composition in the prevention or treatment of oral infections.

16. Use of a system according to any one of the claims 1 to 15, for preparing a hypochlorite composition having a composition pH in the range of 7 to 12, preferably in the range of 7 to 9.5, by combining or mixing at least the first quantity, the second quantity and optionally the amount of pH adjusting agent.

17. Use according to claim 16, wherein the hypochlorite composition is an orally applicable disinfectant for use in the prevention or treatment of oral infections.

18. A hypochlorite composition having a composition pH in the range of 7 to 12, preferably in the range of 7 to 9.5, obtainable or obtained by combining or mixing a first quantity of the first component, a second quantity of the second component and, optionally, an amount of a pH adjusting agent, the first component, the second component and the optional pH adjusting agent being defined as in any one of the claims 1 to 12.

19. The hypochlorite composition as claimed in claim 18, wherein the pH induced thickener comprises or consists of one or more alkali soluble emulsion polymers and/or one or more hydrophobically-modified alkali-soluble emulsion polymers.

20. The hypochlorite composition of claim 16 or 17, having a viscosity higher than 50000 mPa·s, preferably higher than 100000 mPa·s at standard conditions.

21. A method (300) for preparing a hypochlorite composition having a composition pH in the range of 7 to 12, the method comprising combining:
- a first quantity of a first component comprising hypochlorite or a source of hypochlorite and water and having a first pH in the range of 11 to 13.5;
- a second quantity of a second component comprising a pH induced thickener capable of thickening a composition upon an increase of the pH of the composition from acidic to alkaline value, the pH induced thickener being present in at least one of:
- a solvent and/or dispersion medium free form; and
- a solvent and/or dispersion medium containing form in which the pH induced thickener is dissolved and/or dispersed at a second pH lower than 7; and
- optionally, an amount of a pH adjusting agent chosen to cause the hypochlorite composition to have the composition pH;
the hypochlorite composition being obtainable by combining at least the first quantity of the first component, the second quantity of the second component and optionally the amount of pH adjusting agent.
